# EUROPEAN PATENT SPECIFICATION

(11) **EP 2 177 611 B1**
(45) Date of publication and mention of the grant of the patent: **20.11.2013**
(21) Application number: 08790313.4
(22) Date of filing: 30.07.2008
(51) Int. Cl.: C12N 15/09, C07K 16/00, C07K 19/00, C12N 1/15, C12N 1/19, C12N 1/21, C12N 5/10, C12N 9/86, G01N 21/78, G01N 33/53, C07K 16/18, C07K 16/44, G01N 33/542, G01N 33/68, C07K 16/40

(54) **METHOD FOR MEASUREMENT OF CONCENTRATION OF ANTIGEN**
VERFAHREN ZUR MESSUNG DER ANTIGENKONZENTRATION
PROCÉDÉ DE MESURE DE LA CONCENTRATION D'UN ANTIGÈNE

(30) Priority: 01.08.2007 JP 2007201147
(43) Date of publication of application: 21.04.2010
(73) Proprietor: Japan Science and Technology Agency, Kawaguchi-shi Saitama 332-0012 (JP)
(72) Inventor: UEDA, Hiroshi, Kanagawa 226-8503 (JP); KOJIMA, Miki, Kanagawa 226-8503 (JP)
(74) Representative: Wroe, Stephanie
(86) International application number: PCT/JP2008/002049
(87) International publication number: WO 2009/016839

(56) References cited:
- WO-A2-2005/072392
- US-A1- 2003 165 825
- YOKOZEKI T ET AL: "A HOMOGENEOUS NONCOMPETITIVE IMMUNOASSAY FOR THE DETECTION OF SMALL HAPTENS", ANALYTICAL CHEMISTRY, AMERICAN CHEMICAL SOCIETY, US, vol. 74, no. 11, 1 June 2002 (2002-06-01), pages 2500-2504, XP001132315, ISSN: 0003-2700, DOI: 10.1021/AC015743X
- OSUNA J ET AL: "Improving a circularly permuted TEM-1 beta-lactamase by directed evolution", PROTEIN ENGINEERING, OXFORD UNIVERSITY PRESS, SURREY, GB, vol. 15, no. 6, 1 June 2002 (2002-06-01), pages 463-470, XP002506327, ISSN: 0269-2139, DOI: 10.1093/PROTEIN/15.6.463
- YOKOZEKI T. ET AL.: 'A homogeneous noncompetitive immunoassay for the detection of small haptens' ANAL. CHEM. vol. 74, no. 11, 2002, pages 2500 - 2504, XP001132315
- BAIRD G.S. ET AL.: 'Circular permutation and receptor insertion within green fluorescent proteins' PROC. NATL. ACAD. SCI. U.S.A. vol. 96, no. 20, 1999, pages 11241 - 11246, XP002201852
- WEHRMAN T. ET AL.: 'Protein-protein interactions monitored in mammalian cells via complementation of beta - lactamase enzyme fragments' PROC. NATL. ACAD. SCI. U.S.A. vol. 99, no. 6, 2002, pages 3469 - 3474, XP002318574
- UEDA H.: 'Best Couple o Sagase!: Tanpakushitsukan Sogo Sayo o Kenshutsu suru Atarashii Hoho' JOURNAL OF THE SOCIETY FOR BIOSCIENCE AND BIOENGINEERING, JAPAN vol. 80, no. 9, 2002, page 449
- KOJIMA M. ET AL.: 'Kotai - En Junretsu Hen'itai beta Lactamase Konsei Tanpakushitsu o Mochiita Kankyo Osen Busshitsu no Kenshutsu' ABSTRACTS OF ANNUAL MEETING, JAPANESE SOCIETY OF ENZYME ENGINEERING vol. 58TH, 12 October 2007, page 76
- KOJIMA M. ET AL.: 'Kotai Koso Yugo Tanpaku o Mochiita Kankyo Osen Busshitsu Shijikin no Sosei' ABSTRACTS OF THE ANNUAL MEETING OF THE SOCIETY FOR BIOTECHNOLOGY, JAPAN vol. 59TH, 02 August 2007, page 89

### SEQUENCE LISTING

<110> Japan Science and Technology Agency
<120> Method for measurement of concentration of antigen
<130> P039340EP
<140> 08790313.4
   <141> 2008-07-30
<150> JP 2007-201147
   <151> 2007-08-01
<160> 28
<170> PatentIn version 3.1
<210> 1
   <211> 120
   <212> PRT
   <213> Mus musculus
<400> 1
   Gln Val Gln Leu Gln Gln Pro Gly Ala Glu Leu Val Lys Pro Gly Ala
   1               5                   10                  15
   Ser Val Lys Leu Ser Cys Lys Ala Ser Gly Tyr Thr Phe Thr Ser Tyr
               20                  25                  30
   Trp Met His Trp Val Lys Gln Arg Pro Gly Arg Gly Leu Glu Trp Ile
           35                  40                  45
   Gly Arg Ile Asp Pro Asn Ser Gly Gly Thr Lys Tyr Asn Glu Lys Phe
       50                  55                  60
   Lys Ser Lys Ala Thr Leu Thr Val Asp Lys Pro Ser Ser Thr Ala Tyr
   65                  70                  75                  80
   Met Gln Leu Ser Ser Leu Thr Ser Glu Asp Ser Ala Val Tyr Tyr Cys
                   85                  90                  95
   Ala Arg Tyr Asp Tyr Tyr Gly Ser Ser Tyr Phe Asp Tyr Trp Gly Gln
               100                 105                 110
   Gly Thr Thr Leu Thr Val Ser Ser
           115                 120
<210> 2
   <211> 360
   <212> DNA
   <213> Mus musculus
<400> 2
   caggtccaac tgcagcagcc tggggctgag cttgtgaagc ctggggcttc agtgaagctg      60
   tcctgcaagg cttctggcta caccttcacc agctactgga tgcactgggt gaagcagagg     120
   cctggacgag gccttgagtg gattggaagg attgatccta atagtggtgg tactaagtac     180
   aatgagaagt tcaagagcaa ggccacactg actgtagaca aaccctccag cacagcctac     240
   atgcagctca gcagcctgac atctgaggac tctgcggtct attattgtgc aagatacgat     300
   tactacggta gtagctactt tgactactgg ggccaaggca ccactctcac agtctcctca     360
<210> 3
   <211> 111
   <212> PRT
   <213> Mus musculus
<400> 3
   Asp Ile Gln Ala Val Val Thr Gln Glu Ser Ala Leu Thr Thr Ser Pro
   1 5 10 15
   Gly Glu Thr Val Thr Leu Thr Cys Arg Ser Ser Thr Gly Ala Val Thr
   20 25 30
   Thr Ser Asn Tyr Ala Asn Trp Val Gln Glu Lys Pro Asp His Leu Phe
   35 40 45
   Thr Gly Leu Ile Gly Gly Thr Asn Asn Arg Ala Pro Gly Val Pro Ala
   50 55 60
   Arg Phe Ser Gly Ser Leu Ile Gly Asp Lys Ala Ala Leu Thr Ile Thr
   65 70 75 80
   Gly Ala Gln Thr Glu Asp Glu Ala Ile Tyr Phe Cys Ala Leu Trp Tyr
   85 90 95
   Ser Asn His Trp Val Phe Gly Gly Gly Thr Lys Leu Thr Val Leu
   100 105 110
<210> 4
   <211> 333
   <212> DNA
   <213> Mus musculus
<400> 4
   gatatccagg ctgttgtgac tcaggaatct gcactcacca catcacctgg tgaaacagtc 60
   acactcactt gtcgctcaag tactggggct gttacaacta gtaactatgc caactgggtc 120
   caagaaaaac cagatcattt attcactggt ctaataggtg gtaccaacaa ccgagctcca 180
   ggtgttcctg ccagattctc aggctccctg attggagaca aggctgccct caccatcaca 240
   ggggcacaga ctgaggatga ggcaatatat ttctgtgctc tatggtacag caaccattgg 300
   gtgttcggtg gaggaaccaa actgactgtc ctc 333
<210> 5
   <211> 147
   <212> PRT
   <213> Escherichia coli
<400> 5
   His Pro Glu Thr Leu Val Lys Val Lys Asp Ala Glu Asp Gln Leu Gly
   1               5                   10                  15
   Ala Arg Val Gly Tyr Ile Glu Leu Asp Leu Asn Ser Gly Lys Ile Leu
               20                  25                  30
   Glu Ser Phe Arg Pro Glu Glu Arg Phe Pro Met Met Ser Thr Phe Lys
           35                  40                  45
   Val Leu Leu Cys Gly Ala Val Leu Ser Arg Ile Asp Ala Gly Gln Glu
       50                  55                  60
   Gln Leu Gly Arg Arg Ile His Tyr Ser Gln Asn Asp Leu Val Glu Tyr
   65                  70                  75                  80
   Ser Pro Val Thr Glu Lys His Leu Thr Asp Gly Met Thr Val Arg Glu
                   85                  90                  95
   Leu Cys Ser Ala Ala Ile Thr Met Ser Asp Asn Thr Ala Ala Asn Leu
               100                 105                 110
   Leu Leu Thr Thr Ile Gly Gly Pro Lys Glu Leu Thr Ala Phe Leu His
           115                 120                 125
   Asn Met Gly Asp His Val Thr Arg Leu Asp Arg Trp Glu Pro Glu Leu
       130                 135                 140
   Asn Glu Ala
   145
<210> 6
   <211> 441
   <212> DNA
   <213> Escherichia coli
<400> 6
   cacccagaaa cgctggtgaa agtaaaagat gctgaagatc agttgggtgc acgagtgggt 60
   tacatcgaac tggatctcaa cagcggtaag atccttgaga gttttcgccc cgaagaacgt 120
   tttccaatga tgagcacttt taaagttctg ctatgtggcg cggtattatc ccgtattgac 180
   gccgggcaag agcaactcgg tcgccgcata cactattctc agaatgactt ggttgagtac 240
   tcaccagtca cagaaaagca tcttacggat ggcatgacag taagagaatt atgcagtgct 300
   gccataacca tgagtgataa cactgcggcc aacttacttc tgacaacgat cggaggaccg 360
   aaggagctaa ccgctttttt gcacaacatg ggggatcatg taactcgcct tgatcgttgg 420
   gaaccggagc tgaatgaagc c 441
<210> 7
   <211> 119
   <212> PRT
   <213> Escherichia coli
<400> 7
   Asn Glu Ala Ile Pro Asn Asp Glu Arg Asp Thr Thr Met Pro Ala Ala
   1               5                   10                  15
   Met Ala Thr Thr Leu Arg Lys Leu Leu Thr Gly Glu Leu Leu Thr Leu
               20                  25                  30
   Ala Ser Arg Gln Gln Leu Ile Asp Trp Met Glu Ala Asp Lys Val Ala
           35                  40                  45
   Gly Pro Leu Leu Arg Ser Ala Leu Pro Ala Gly Trp Phe Ile Ala Asp
       50                  55                  60
   Lys Ser Gly Ala Gly Glu Arg Gly Ser Arg Gly Ile Ile Ala Ala Leu
   65                  70                  75                  80
   Gly Pro Asp Gly Lys Pro Ser Arg Ile Val Val Ile Tyr Thr Thr Gly
                   85                  90                  95
   Ser Gln Ala Thr Met Asp Glu Arg Asn Arg Gln Ile Ala Glu Ile Gly
               100                 105                 110
   Ala Ser Leu Ile Lys His Trp
           115
<210> 8
   <211> 357
   <212> DNA
   <213> Escherichia coli
<400> 8
   aatgaagcca taccaaacga cgagcgtgac accacgatgc ctgcagcaat ggcaacaacg     60
   ttgcgcaaac tattaactgg cgaactactt actctagctt cccggcaaca attaatagac    120
   tggatggagg cggataaagt tgcaggacca cttctgcgct cggcccttcc ggctggctgg    180
   tttattgctg ataaatctgg agccggtgag cgtgggtctc gcggtatcat tgcagcactg    240
   gggccagatg gtaagccctc ccgtatcgta gttatctaca cgacggggag tcaggcaact    300
   atggatgaac gaaatagaca gatcgctgag ataggtgcct cactgattaa gcattgg       357
<210> 9
   <211> 36
   <212> DNA
   <213> Artificial sequence
<220>
   <223> primer
<400> 9
   cattgggaca agagccaccc agaaacgctg gtgaaa 36
<210> 10
   <211> 30
   <212> DNA
   <213> Artificial sequence
<220>
   <223> primer
<400> 10
   ggcgatatcg gcttcattca gctccggttc 30
<210> 11
   <211> 27
   <212> DNA
   <213> Artificial sequence
<220>
   <223> primer
<400> 11
   ggcgatatca atgaagccat accaaac 27
<210> 12
   <211> 33
   <212> DNA
   <213> Artificial sequence
<220>
   <223> primer
<400> 12
   tgggtggctc ttgtcccaat gcttaatcag tga 33
<210> 13
   <211> 269
   <212> PRT
   <213> Escherichia coli
<400> 13
   Asn Glu Ala Ile Pro Asn Asp Glu Arg Asp Thr Thr Met Pro Ala Ala
   1               5                   10                  15
   Met Ala Thr Thr Leu Arg Lys Leu Leu Thr Gly Glu Leu Leu Thr Leu
               20                  25                  30
   Ala Ser Arg Gln Gln Leu Ile Asp Trp Met Glu Ala Asp Lys Val Ala
           35                  40                  45
   Gly Pro Leu Leu Arg Ser Ala Leu Pro Ala Gly Trp Phe Ile Ala Asp
       50                  55                  60
   Lys Ser Gly Ala Gly Glu Arg Gly Ser Arg Gly Ile Ile Ala Ala Leu
   65                  70                  75                  80
   Gly Pro Asp Gly Lys Pro Ser Arg Ile Val Val Ile Tyr Thr Thr Gly
                   85                  90                  95
   Ser Gln Ala Thr Met Asp Glu Arg Asn Arg Gln Ile Ala Glu Ile Gly
               100                 105                 110
   Ala Ser Leu Ile Lys His Trp Asp Lys Ser His Pro Glu Thr Leu Val
           115                 120                 125
   Lys Val Lys Asp Ala Glu Asp Gln Leu Gly Ala Arg Val Gly Tyr Ile
       130                 135                 140
   Glu Leu Asp Leu Asn Ser Gly Lys Ile Leu Glu Ser Phe Arg Pro Glu
   145                 150                 155                 160
   Glu Arg Phe Pro Met Met Ser Thr Phe Lys Val Leu Leu Cys Gly Ala
                   165                 170                 175
   Val Leu Ser Arg Ile Asp Ala Gly Gln Glu Gln Leu Gly Arg Arg Ile
               180                 185                 190
   His Tyr Ser Gln Asn Asp Leu Val Glu Tyr Ser Pro Val Thr Glu Lys
           195                 200                 205
   His Leu Thr Asp Gly Met Thr Val Arg Glu Leu Cys Ser Ala Ala Ile
       210                 215                 220
   Thr Met Ser Asp Asn Thr Ala Ala Asn Leu Leu Leu Thr Thr Ile Gly
   225                 230                 235                 240
   Gly Pro Lys Glu Leu Thr Ala Phe Leu His Asn Met Gly Asp His Val
                   245                 250                 255
   Thr Arg Leu Asp Arg Trp Glu Pro Glu Leu Asn Glu Ala
               260                 265
<210> 14
   <211> 807
   <212> DNA
   <213> Escherichia coli
<400> 14
   aatgaagcca taccaaacga cgagcgtgac accacgatgc ctgcagcaat ggcaacaacg      60
   ttgcgcaaac tattaactgg cgaactactt actctagctt cccggcaaca attaatagac 120
   tggatggagg cggataaagt tgcaggacca cttctgcgct cggcccttcc ggctggctgg 180
   tttattgctg ataaatctgg agccggtgag cgtgggtctc gcggtatcat tgcagcactg 240
   gggccagatg gtaagccctc ccgtatcgta gttatctaca cgacggggag tcaggcaact 300
   atggatgaac gaaatagaca gatcgctgag ataggtgcct cactgattaa gcattgggac 360
   aagagccacc cagaaacgct ggtgaaagta aaagatgctg aagatcagtt gggtgcacga 420
   gtgggttaca tcgaactgga tctcaacagc ggtaagatcc ttgagagttt tcgccccgaa 480
   gaacgttttc caatgatgag cacttttaaa gttctgctat gtggcgcggt attatcccgt 540
   attgacgccg ggcaagagca actcggtcgc cgcatacact attctcagaa tgacttggtt 600
   gagtactcac cagtcacaga aaagcatctt acggatggca tgacagtaag agaattatgc 660
   agtgctgcca taaccatgag tgataacact gcggccaact tacttctgac aacgatcgga 720
   ggaccgaagg agctaaccgc ttttttgcac aacatggggg atcatgtaac tcgccttgat 780
   cgttgggaac cggagctgaa tgaagcc 807
<210> 15
   <211> 42
   <212> DNA
   <213> Artificial sequence
<220>
   <223> Dralrev
<400> 15
   ggtggaggcg gttcaggctt taaaggcagt ggcggtggcg gg 42
<210> 16
   <211> 42
   <212> DNA
   <213> Artificial sequence
<220>
   <223> Dralfor
<400> 16
   cccgccaccg ccactgcctt taaagcctga accgcctcca cc 42
<210> 17
   <211> 46
   <212> DNA
   <213> Artificial sequence
<220>
   <223> BLAsigBack
<400> 17
   tcaccatcac taagaaggag atatcatatg agtattcaac atttcc 46
<210> 18
   <211> 20
   <212> DNA
   <213> Artificial sequence
<220>
   <223> BLAdraFor
<400> 18
   cggcgaccga gttgctcttg 20
<210> 19
   <211> 20
   <212> DNA
   <213> Artificial sequence
<220>
   <223> BLAbsaBack
<400> 19
   atggaggcgg ataaagttgc 20
<210> 20
   <211> 47
   <212> DNA
   <213> Artificial sequence
<220>
   <223> BLAHisFor
<400> 20
   ccttcttagt gatggtgatg atgatgccaa tgcttaatca gtgaggc 47
<210> 21
   <211> 30
   <212> DNA
   <213> Artificial sequence
<220>
   <223> BLA168Ncorev
<400> 21
   catgccatgg gcaatgaagc cataccaaac 30
<210> 22
   <211> 30
   <212> DNA
   <213> Artificial sequence
<220>
   <223> BLA170Xhofor
<400> 22
   ccgctcgagg gcttcattca gctccggttc 30
<210> 23
   <211> 42
   <212> DNA
   <213> Artificial sequence
<220>
   <223> MKback2A
<400> 23
   gcgcaagctc agtcgacgga yattgtgmts acmcarwctm ca 42
<210> 24
   <211> 30
   <212> DNA
   <213> Artificial sequence
<220>
   <223> VkNotFor
<400> 24
   atggtgctcg actgcggccg cccgttttat 30
<210> 25
   <211> 27
   <212> DNA
   <213> Artificial sequence
<220>
   <223> NotdXback
<400> 25
   gcagtcgagc accatcacca ccaccac 27
<210> 26
   <211> 20
   <212> DNA
   <213> Artificial sequence
<220>
   <223> pETDra3For
<400> 26
   tgagtgttgt tccagtttgg 20
<210> 27
   <211> 17
   <212> DNA
   <213> Artificial sequence
<220>
   <223> M13RV
<400> 27
   caggaaacag ctatgac 17
<210> 28
   <211> 27
   <212> DNA
   <213> Artificial sequence
<220>
   <223> JH1
<400> 28
   actgctcgag acggtgaccg tggtccc 27

## Description

### Technical Field

The present invention relates to an antibody-enzyme fusion protein and a method for detecting an antigen using the antibody-enzyme fusion protein.

### Background Art

As immunoassays using antibodies, such methods as an enzyme-linked immunosorbent assay (ELISA) and the like are widely used. However, when the antigen has a low molecular weight, there was a problem such as the sensitivity of ELISA and the like decreases for a low molecular antigen, firstly because the interface area between an antigen-binding site (paratope) of the antibody and the antigen becomes small, and secondly the sandwich assay with excellent sensitivity cannot be employed because of the too small antigen, and the measurement has to be conducted by a competitive assay. Therefore, methods that can also measure low molecular weight antigens with higher sensitivity in a noncompetitive manner are being developed.

For example, nonpatent document 1 discloses a method for detecting 4-hydroxy-3-nitrophencetyl (NP), a low molecular weight antigen, with high sensitivity, which method comprises use of a fusion protein that consists of a V_{H} domain polypeptide of B1-8 antibody that binds NP, and an α fragment of E. coli β- galactosidase (a V_{H} domain polypeptide linked to the N-terminus of the α fragment), and a fusion protein that consists of a V_{L} domain polypeptide of the above-mentioned antibody and an ω fragment of E. coli β-galactosidase (a V_{L} domain polypeptide linked to the N-terminus of the ω fragment) . In this method, the detection of NP antigen is conducted by detecting the increase of β-galactosidase activity (so-called activity complementation) which results from the interaction of V_{H} domain polypeptide in one fusion protein and the V_{L} domain polypeptide in the other fusion protein via an NP antigen, and the subsequent binding of the α fragment with the ω fragment in both fusion proteins. Also, the method enabled to measure the low molecular antigen with higher sensitivity than the conventional ELISA. In addition, the method was advantageous over the conventional ELISA in that it is a measurement method called homogeneous assay, that can measure without any operations of washing unbound labeled secondary antibodies (in non-competitive assays) or labeled antigens (in competitive assays) unlike the typical ELISA, and that the automation of a measuring device is easy, resulting in a quick measurement. However, there was a room for improvement with regard to simplicity of detection, efficiency, stability, and the like requiring that the concentration of both fusion proteins to be used should be set precisely and the like.

Nonpatent document 1: Anal. Chem., 74, 2500-2504 (2002)

### Disclosure of the Invention

### Object to be solved by the present invention

The present invention improves the aforementioned back ground art, and the obj ect of the present invention is to provide a method for detecting an antigen even when the target antigen is a low molecular weight antigen, in a simpler, more efficient and more stable manner and with higher sensitivity.

### Means to solve the object

The present inventors have found that by tethering a fusion protein comprising a V_{H} domain polypeptide and one part of an enzyme protein and a fusion protein comprising a V_{L} domain polypeptide and the remaining part of the enzyme protein with a linker peptide, the drawbacks of the method of the nonpatent document 1 can be remarkably improved. In other words, the present inventors have found that when an ICP antigen with an extremely low concentration is allowed to contact with E. coli expressing a fusion protein comprising a V_{H} domain polypeptide of an antibody that recognizes imidacloprid (ICP), a polypeptide being a part of β-lactamase (BLA), a linker peptide, a polypeptide being another part of the BLA and a V_{L} domain polypeptide of the antibody in this order, the BLA activity within E. coli is elevated depending on the ICP antigen concentration and with remarkably higher sensitivity compared to the molecule without linker peptide. The present invention has been thus completed.

Specifically, the present invention relates to (1) a fusion protein comprised of a polypeptide containing a V_{H} domain of an antibody; a given protein; a linker peptide; a partner protein having a binding activity to the protein and capable of detecting the presence or absence of the binding to the protein; and a polypeptide containing a V_{L} domain of the antibody; in this order or in reverse order, wherein the presence or absence of the binding of the polypeptide containing a V_{H} domain and the polypeptide containing a V_{L} domain to the antigen induces the presence or absence of the binding of the protein and the partner protein; (2) the fusion protein according to (1), wherein the combination of the given protein and the partner protein is a combination of a protein containing an N-terminal fragment of an enzyme protein and a protein containing a C-terminal fragment of the enzyme protein, and that the presence or absence of the binding of the protein containing the N-terminal fragment and the protein containing the C-terminal fragment can be detected by the change of the given enzyme activity of the enzyme protein; (3) the fusion protein according to (2), wherein the enzyme protein is β-lactamase; (4) the fusion protein according to (3), wherein the protein containing an N-terminal fragment of β-lactamase is a protein consisted of the amino acid sequence represented by SEQ ID NO: 5, and the protein containing a C-terminal fragment of β-lactamase is a protein consisted of the amino acid sequence represented by SEQ ID NO: 7; (5) the fusion protein according to any one of (1) to (4), wherein the linker peptide is the amino acid sequence consisted of Asp-Lys-Ser; (6) the fusion protein according to any one of (1) to (5), wherein the sequence of a given protein; a linker peptide; and a partner protein is a protein consisted of the amino acid sequence represented by SEQ ID NO: 13; (7) a DNA encoding the fusion protein according to any one of (1) to (6); (8) a recombinant vector having the DNA according to (7); (9) a transformed cell transformed by the recombinant vector according to (8); (10) a method for detecting an antigen in a sample, comprising the steps of: contacting the sample with the fusion protein according to any one of claims 1 to 6 in which a polypeptide containing a V_{H} domain and a polypeptide containing a V_{L} domain can bind to the antigen, or with the transformed cell according to claim 9; and detecting the binding of a partner protein and a given protein in the fusion protein or in a fusion protein in which the transformed cell is expressed; and (11) a kit for detecting an antigen provided with the fusion protein according to any one of (1) to (6) which is a protein wherein a given enzyme activity exhibited by a given protein and/or a partner protein changes when the given protein and the partner protein are bound to each other compared to when they are not bound, or the transformed cell according to claim 9; and a chromogenic substrate or a fluorogenic substrate that can be detected due to the change of the enzyme activity. In addition, the sequences in the present specification are written from the 5' end to 3'end.

### Brief Description of Drawings

[Fig. 1]
   It is a schematic diagram of a conformational change of a fusion protein of the present invention depending on the presence or absence of the binding of the V_{H} domain polypeptide and the V_{L} domain polypeptide to an antigen.
[Fig. 2]
   It is a drawing showing the construct of the antibody-circularly permutated BLA fusion protein expression vector of the present invention, and a construct used for preparing the vector.
[Fig. 3]
   It is a drawing showing the construct of an antibody-linker lacking circularly permutated BLA fusion protein expression vector.
[Fig. 4]
   It is a drawing showing a construct used for preparing an antibody-linker lacking circularly permutated BLA fusion protein expression vector, and an outline of the method for preparing the construct.
[Fig. 5]
   It is a drawing showing the construct of a circularly permutated BLA fusion protein expression vector.
[Fig. 6]
   It is a drawing showing the growth of E. coli in the presence or absence of ICP.
[Fig. 7]
   It is a drawing showing the growth of a V_{H}-cpBLA-V_{L}/pET 26/BL21 strain in the presence or absence of ampicillin, and in the presence or absence of ICP or TCP.
[Fig. 8]
   It is a drawing showing the growth of V_{H}-split-cpBLA -V_{L}/pET26/C43 strain in the presence or absence of ampicillin, and in the presence or absence of ICP or TCP.
[Fig. 9]
   It is a drawing showing the results of Western blotting using bacteria of a V_{H}-cpBLA-V_{L}/pET26/BL21 strain cultured in the presence or absence of an antigen (TCP or ICP).
[Fig. 10]
   It is a drawing showing the results of SDS-PAGE of a protein partially purified from a V_{H}-cpBLA-V_{L}/pET26/BL21 strain.
[Fig. 11]
   It is a drawing showing the ICP binding activity of a purified antibody-enzyme fusion protein (V_{H}-cpBLA-V_{L}).
[Fig. 12]
   It is a drawing showing a change in the activity of a purified antibody-enzyme fusion protein (V_{H}-cpBLA-V_{L}) by adding ICP/TCP using Nitrocefin as a substrate.
[Fig. 13]
   It is a drawing that shows a change in the activity of a purified antibody-enzyme fusion protein (V_{H}-cpBLA-V_{L}) by adding ICP/TCP using Fluorocillin as a substrate.
[Fig. 14]
   It is a drawing that shows a change in an activity of a purified antibody-enzyme fusion protein (V_{H}-cpBLA-V_{L} (219)) by adding BGP-C7 peptide using Fluorocillin as a substrate.
[Fig. 15]
   It is a drawing that shows a change in an activity of a purified antibody-enzyme fusion protein (V_{H}-cpBLA-V_{L} (219)) by adding each kind of BGP peptide/ICP/TCP using Fluorocillin as a substrate.

### Best Mode of Carrying Out the Invention

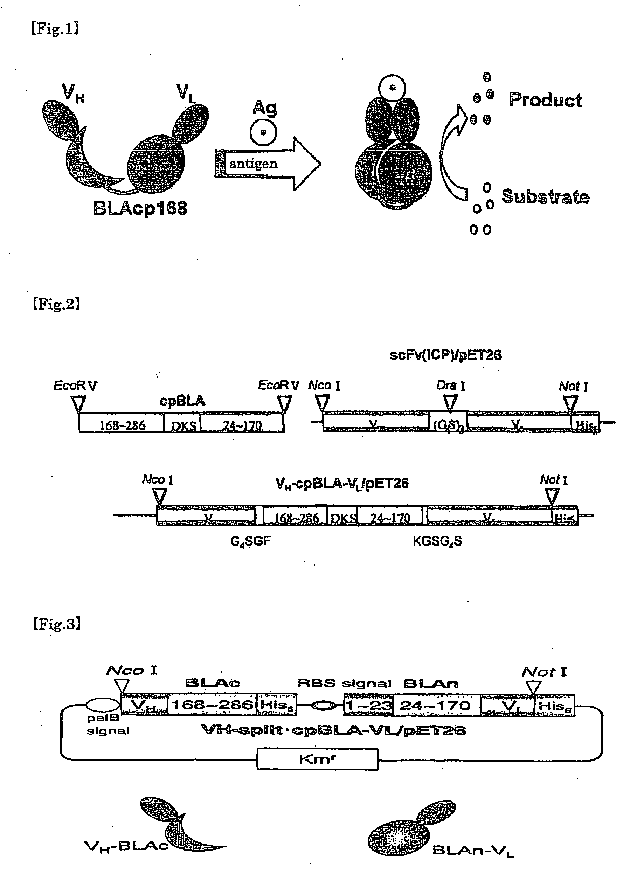

As for a fusion protein of the present invention, it is not specially limited as long as it is a fusion protein comprised of a polypeptide containing a V_{H} domain of an antibody; a given protein; a linker peptide; a partner protein having a binding activity to the protein and capable of detecting the presence or absence of the binding to the protein; and a polypeptide containing a V_{L} domain of the antibody; in this order or in reverse order, wherein the presence or absence of the binding of the polypeptide containing a V_{H} domain and the polypeptide containing a V_{L} domain to the antigen induces the presence or absence of the binding of the protein and the partner protein. A schematic diagram of the structural change of the fusion protein of the present invention depending on the presence or absence of the binding of the polypeptide containing a V_{H} domain and the polypeptide containing a V_{L} domain to the antigen is shown in Fig.1.

As for a polypeptide containing a V_{H} domain (hereinafter also referred to as "V_{H} domain polypeptide") or a polypeptide containing a V_{L} domain (hereinafter also referred to as "V_{L} domain polypeptide") used for the fusion protein of the present invention, it is not specially limited as long as it is a V_{H} domain polypeptide and a V_{L} domain polypeptide of an antibody that specifically binds to a given antigen, and that the presence or absence of the binding of the given protein and the partner protein is induced by the presence or absence of the binding of the V_{H} domain polypeptide and the V_{L} domain polypeptide to the antigen, when used for the fusion protein of the present invention. For example, the V_{H} domain polypeptide (amino acid sequence: SEQ ID NO: 1; nucleotide sequence: SEQ ID NO: 2) or the V_{L} domain polypeptide (amino acid sequence: SEQ ID NO: 3; nucleotide sequence: SEQ ID NO: 4) of a B1-8 antibody that binds to 4-hydroxy-3-nitrophencetyl (NP), the V_{H} domain polypeptide or the V_{L} domain polypeptide of HyHEL-10 that binds to lysozyme (Nat. Biotechnol., 14, 1714-1718 (1996); J. Immunol. Methods, 224, 171-184 (1999)), the V_{H} domain polypeptide or the V_{L} domain polypeptide of an antibody that binds to imidacloprid (ICP) (33C3-1-1, monoclonal antibody produced from hybridoma deposited as FERM P-17094), Japanese Laid-open Patent Application No. 2000-191698), the V_{H} domain polypeptide or the V_{L} domain polypeptide of the antibody KTM219 that binds to osteocalcin C-terminal peptide (Anal. Chem. 79, 6197(2007) can be preferably exemplified.
In addition, as for the V_{H} domain polypeptide or the V_{L} domain polypeptide used for the fusion protein of the present invention, the polypeptide consisting of the V_{H} domain or the polypeptide consisting of the V_{L} domain can be preferably exemplified. Further, as long as it specifically binds to a given antigen, a polypeptide in which a mutation, insertion, or deletion has been introduced into a part of the V_{H} domain, or a polypeptide in which a mutation, insertion, or deletion has been introduced into a part of the V_{L} domain can be used. Although the mutation, insertion, or deletion is not specially limited, it is preferably a mutation, insertion, or deletion of 10 or less amino acids, more preferably 5 or less amino acids, further preferably 3 or less amino acids, and even further preferably 1 or less amino acid.

The preparation method of the V_{H} domain polypeptide or the V_{L} domain polypeptide used for the fusion protein of the present invention is not specially limited, and it may be prepared from an antibody whose sequence is publicly known. Alternatively, the V_{H} domain polypeptide or the V_{L} domain polypeptide may be prepared by producing an antibody against the target antigen using commonly used protocols (e.g., see Japanese Translation of PCT International Publication No.2006-523088), followed by, for example, cloning etc. a DNA sequence encoding the V_{H} domain polypeptide or the V_{L} domain polypeptide of the antibody or the like to obtain the polypeptide to be used for the fusion protein of the present invention, or the DNA sequence encoding the polypeptide, and by expressing the DNA sequence in appropriate cells or the like. According to such methods, the DNA sequence encoding the V_{H} domain polypeptide or the V_{L} domain polypeptide that can specifically binds to the intended antigen which is a detection target can be obtained easily. For example, the above-mentioned Japanese Translation of PCT International Publication No.2006-523088 describes an antibody that specifically binds to human IL-8 or the sequence of the V_{H} domain and V_{L} domain. In addition, with the assays described in the following Examples, it can be confirmed easily whether the prepared V_{H} domain polypeptide or V_{L} domain polypeptide can be suitably used for the fusion protein of the present invention.

The given protein or its partner protein used for the fusion protein of the present invention (hereinafter also referred together to as "both proteins") are not specially limited as long as the partner protein has a binding activity to the proteins and the presence or absence of the binding to the proteins can be detected, and that the presence or absence of the binding of the V_{H} domain polypeptide and the V_{L} domain polypeptide to the antigen in the fusion protein of the present invention induces the presence or absence of the binding of the protein and the partner protein. However, a combination that can easily detect the presence or absence of the binding of both proteins can be preferably exemplified, and examples include a combination in which a given enzyme activity exhibited by the given protein and/or partner protein changes (increases, lowers, expresses, or extincts) when both proteins are bound to each other compared to when they are not bound (in particular, a combination that can visually detect the change of the enzyme activity with a chromogenic substrate or a fluorogenic substrate). Among them, a combination comprising a protein containing a part of an enzyme protein and a protein containing the remaining part of the enzyme protein, wherein the enzyme activity of the enzyme changes (in particular, increases or expresses) when both proteins are bound to each other, can be more preferably exemplified. Further, a combination comprising a protein containing an N-terminal fragment of the specific enzyme and a protein containing the C-terminal fragment wherein the enzyme activity of the enzyme changes (in particular, increases or expresses) when both proteins are bound to each other can be further more preferably exemplified. Further, a combination of the α fragment of β-galactosidase derived from E. coli and the ω fragment thereof, and the protein consisted of the amino acid sequence at positions 24 - 170 of β-lactamase (BLA) derived from E. coli (amino acid sequence: SEQ ID NO: 5; nucleotide sequence: SEQ ID No: 7) and the protein consisted of the amino acid sequence at positions 168 - 286 (amino acid sequence: SEQ ID NO: 7; nucleotide sequence: SEQ ID No: 8) can be most preferably exemplified. In addition, the names of the given protein and the partner protein in the present invention are distinguished only as a matter of convenience, and for example, the protein consisted of the amino acid sequence at positions 24 - 170 of the BLA may be the given protein, and the protein consisted of the amino acid sequence at positions 168 - 286 of the BLA may be the partner protein, while the protein consisted of the amino acid sequence at positions 168 - 286 of the BLA may be the given protein, and the protein consisted of the amino acid sequence at positions 24 - 170 of the BLA may be the partner protein.

In the case where the both proteins of the present invention are a combination of the α fragment and the ω fragment of β-galactosidase derived from E. coli, and when the both fragments are bound to each other, a so-called activity complementation arises, and the β-galactosidase activity increases. The increase in the β-galactosidase activity can be detected with high sensitivity by adding chemiluminecsent substrates such as Galacton-Plus (registered trademark) (Applied Biosystems Inc.). In the case of a combination of a protein consisted of the amino acid sequence at positions 24 - 170 and a protein consisted of the amino acid sequence at positions 168 - 286 of β-lactamase (BLA) derived from E.coli, when both of these proteins are bound to each other, the β-lactamase activity increases. The increase in the β-lactamase activity can be detected with high sensitivity by adding chromogenic substrates such as Nitrocefin (Nature Biotechnol., 20, 619-622 (2002)), or fluorogenic substrates such as Fluorocillin Green 495/525 beta lactamase substrate (Invitrogen Inc.).

As other combinations that can to be applied as the two proteins of the present invention, examples include a combination of the N-terminal fragment and the C-terminal fragment of luciferase, in which the luciferase activity is restored when both fragments are bound to each other (Anal Chem. , 75, 1584-1589 (2003)); a combination of a fragment of up to position 59 and a fragment at position and after of Cre enzyme, in which the Cre activity is restored when both fragments are bound to each other (Nucleic Acids Research, 31, e131 (2003)); a combination of the N-terminal fragment and the C-terminal fragment of dihydrofolate reductase, in which the dihydrofolate reductase activity is restored when both fragments are bound to each other (Proc. Natl. Acad. Sci. USA, 95, 12141-12146 (1998)); and a combination of a circularly permutant of GFP in which the C-terminus of the C-terminal fragment and the N-terminus of the N-terminal fragment are linked by an insertion sequence (linker sequence) by exchanging the positions of the N-terminal fragment and the C-terminal fragment of GFP (Proc. Natl. Acad. Sci. USA, 96, 12141-12146 (1998)).

As for the two proteins comprising the fusion proteins of the present invention, any publicly known enzyme protein and the like can be used other than the above-mentioned proteins. A combination of two fragments of a given enzyme that expresses no or weak enzyme activity when both fragments are not bound each other, while expresses or increases the enzyme activity (complement the activity) when both fragments are bound to each other can be easily produced by publicly known protein-fragment complementation assays (Nature Biotechnol., 20, 619-622 (2002), or USP 7,160,691). Further, the circular permutant in which both fragments that are able to complement are linked by a linker sequence can also be produced easily based on the above-mentioned document (Proc. Natl. Acid. Sci. USA, 96, 11241-11246 (1999)), etc.
In addition, the sequences of both fragments that complement the activity may be partially overlapped.

The linker peptide used for the fusion protein of the present invention is not specially limited as long as it does not inhibit the binding of a given protein used in the present invention and its partner protein or disturb the detection of the presence or absence of the binding, when the given protein and its partner protein are linked by a linker peptide. However, in order to detect the target antigen using the fusion protein of the present invention with a higher sensitivity, it is preferred to adjust appropriately the length of the linker peptide. When a combination of a protein consisted of the amino acid sequences at positions 24 - 170 (N-terminal fragment) and a protein consisted of the amino acid sequences at positions 168 - 286 (C-terminal fragment) of β-lactamase (BLA) derived from E. coli are used as a given protein and its partner protein of the present invention, the amino acid sequence consisted of Asp-Lys-Ser (DKS) (nucleotide sequence: gacaagagc) can be preferably exemplified as its linker peptide. The preferred number of amino acids of the linker peptide can be readily checked with the assays described in the following Examples regarding a plurality of samples in which the number of amino acids of the linker peptide has been varied.

As for the fusion protein of the present invention, as long as it does not inhibit the binding of the V_{H} domain polypeptide and the V_{L} domain polypeptide to the antigen, the binding of a given protein and its partner protein, and the detection of the binding of a given protein and its partner protein, it is preferred that the fusion protein comprises a polypeptide such as a Tag sequence for purification including His-Tag, besides a V_{H} domain polypeptide of the antibody; a given protein; a linker peptide; a partner protein having a binding activity to the protein and capable of detecting the presence or absence of the binding to the protein, for an easy purification.

As for the DNA encoding the fusion protein of the present invention, it is not specially limited as long as it is a DNA encoding the above-mentioned fusion protein of the present invention, and it can be constructed by positioning the DNA sequences of the V_{H} domain polypeptide, given protein, linker peptide, partner protein, and V_{L} domain polypeptide, in this order with commonly used protocols. As for the DNA encoding the fusion protein of the present invention, specifically, an NcoI-NotI fragment of the V_{H}-cpBLA-V_{L}/pET26 vector can be preferably exemplified.

As for the recombinant vector of the present invention, it is not specially limited as long as it comprises the DNA encoding encodes the above-mentioned fusion protein of the present invention and that it can express the fusion protein of the present invention, the V_{H}-cpBLA-V_{L}/pET26 vector can be preferably exemplified.
The recombinant vector of the present invention can be constructed by appropriately integrating the DNA of the present invention into an expression vector. As such expression vectors, a vector that can self-replicate in a host cell, or a vector that can be inserted into the chromosome of a host cell is preferred, and a vectors comprising a regulatory sequence such as promoters, enhancers and terminators at the positions where the regulatory sequence can express the genes of the present invention can be suitably used.

Among the above-mentioned expression vectors, examples of bacterial expression vectors include pBTrP2, pBTac1, pBTac2 (all from Roche Diagnostics K.K.), pKK233-2 (GE Healthcare Japan Corporation), pSE280(Invitrogen Inc.), pGEMEX-1 (Promega Corporation), pQE-8(QIAGEN K.K.), pQE-30(QIAGEN K.K.), pKYP10 (Japanese Laid-Open Patent Application NO. 58-110600), pKYP200 [Agrc. Biol. Chem., 48, 669 (1984)], pLSA 1 [Agrc. Biol.Chem., 53, 277 (1989)], pGEL1 [Proc. Natl. Acad. Sci. USA, 82, 4306 (1985)], pTP5, pC194, pUC18 [Gene, 33, 103 (1985)], pUC19 [Gene, 33, 103 (1985)], pSTV28 (TAKARABIO INC.), pSTV29 (TAKARA BIO INC.). Examples of bacterial promoters include promoters derived from E. coli, phage or the like such as trp promoters (Ptrp), lac promoters (Plac), PL promoters, PR promoters, and PSE promoters; SP01 promotors, SP02 promotors, penP promotors and the like.

As for the transformed cell of the present invention, it is not specially limited as long as it is a transformed cell introduced with the recombinant vector of the present invention and that can express the fusion protein of the present invention, and V_{H}-cpBLA-V_{L}/pET26/BL21 strain that can be obtained by introducing V_{H}-cpBLA-V_{L}/pET26 recombinant vector of the present invention into E. coli BL21 (DE3, pLysS) strain can be preferably exemplified. As for the method for introducing the recombinant vector of the present invention into host cells when producing the transformed cells, it can be performed according to methods as described in many of the standard laboratory manuals including Davis et al. (BASIC METHODS IN MOLECULAR BIOLOGY, 1986) and Sambrook et al. (MOLECULAR CLONING: A LABORATORY MANUAL, 2nd Ed., Cold Spring Harbor Laboratory Press, Cold Spring Harbor, N.Y., 1989); examples include calcium phosphate transfection, DEAE-dextran-mediated transfection, transvection, microinjection, cationic lipid-mediated transfection, electroporation and genetic transduction. Further, examples of host cells include bacterial cell, yeast cell and animal cells, where E. coli such as the XL10-Gold strain and the BL21 strain, bacteria such as streptomyces, Bacillus subtilis, Streptococcus and Staphylococcus can be preferably exemplified, and E. coli can be particularly preferably exemplified.

As for the method for detecting an antigen of the present invention, it is not specially limited as long as it is a method for detecting an antigen in a sample, comprising the steps of: contacting the sample with the fusion protein of the present invention in which a V_{H} domain polypeptide and a V_{L} domain polypeptide can bind to the antigen, or with the transformed cell of the present invention; and detecting the binding of a given protein and a partner protein in the fusion protein, or in a fusion protein in which the transformed cell is expressed. As for the antigen, it is not specially limited, but when the antigen is a small compound with a molecular weight less than 1000, the advantage of the method for detecting an antigen of the present invention can be particularly attained. Examples of the above-mentioned small compound include neonicotinoid pesticides such as imidacloprid, environmental pollutants such as polychlorinated biphenyl and bisphenol A, poisonous materials such as mycotoxin, and biological materials such as osteocalcin peptides. Since the method for detecting an antigen of the present invention can detect these materials as antigens, it can be advantageously applied to clinical diagnosis of diseases, toxicity test of food products, environmental analysis and the like.
In addition, unlike the typical ELISA method, since the method for detecting an antigen of the present invention does not require the washing process after contacting the sample with the fusion protein of the present invention and the like, the detection of the antigen can be made extremely simply and quickly.

The contact of the fusion protein or the transformed cell of the present invention with a sample can be made, for example, by suspending or diluting the fusion protein or the transformed cell of the present invention and the sample in an appropriate solvent. However, when the transformed cell of the present invention is used directly, it is preferred to use the transformed cell after confirming that the target antigen is ingested within the transformed cell, or the fusion protein of the present invention in which the transformed cell expressed is secreted out of the cell, since it is necessary that the fusion protein of the present invention in which the transformed cell is expressed and the target antigen can be contacted. On the other hand, when the transformed cell of the present invention is used by being disrupted, it is not particularly necessary to confirm this point.
In addition, in the method for detecting an antigen of the present invention, it is preferred to adjust appropriately the concentration of the fusion protein and the transformed cell of the present invention to be used in order to obtain a higher detection sensitivity. The above-mentioned concentration for obtaining a higher sensitivity cannot be described categorically since it differs depending on the kinds of the fusion protein or transformed cell, however, the concentration can be set readily by performing experiments by changing gradually the above-mentioned concentration. However, since the fusion protein of the present invention comprises a linker peptide, a higher detection sensitivity can be obtained with the method for detecting an antigen of the present invention compared with the method of nonpatent document 1.

The detection of the binding of the above given protein and the partner protein can be made in a simple manner and with high sensitivity by, for example, detecting the change (increase, lowering, expression, or extinction) in the given enzyme activity exhibited by the protein and/or the partner protein, and more preferably, by detecting the increase or expression of the given enzyme activity. The detection of the change in the given enzyme activity can be made, for example, by allowing the reaction system to contain a substrate of the enzyme, thereby detecting the change in the amount of substrate after a certain time period. However, from the viewpoint of detecting in a simpler manner and with higher sensitivity, it is preferred to use chromogenic substrates, fluorogenic substrates or the like that enable visual detection of the change in the enzyme activity. Specifically, when a given protein and a partner protein is a combination of the α fragment and the ω fragment of β-galactosidase, by using chemiluminescent substrates such as Galacton-Plus (registered trademark) (Applied Biosystems Inc.), the expression or increase of the β-galactosidase activity can be detected by light emission or an increase of the amount of light emission. Further, when it is a combination of a given protein and a partner protein consisted of the amino acid sequence at positions 24 - 170 of β-lactamase (BLA) (N-terminal fragment) and a protein consisted of the amino acid sequence at positions 168 - 286 (C-terminal fragment), by using chromogenic substrates such as Nitrocefin or fluorogenic substrates such as CCF2/AM and Fluorocillin Green 495/525 beta lactamase substrate (Invitrogen Inc.), the expression or increase of the β-lactamase activity can be detected by coloring or light emission, or by the increase the amount of the coloring or luminescence. The presence or absence of coloring or light emission, or the amount of coloring or light emission can be detected with high sensitivity by using a laser microscope, and the like.

When the transformed cell of the present invention is used in the method for detecting an antigen of the present invention, the detection of the binding of the given protein and the partner protein can also be made at a lower price and in a preferred manner by inspecting the growth rate of the transformed cells. For example, when a given protein and a partner protein are a combination of the protein consisted of the amino acid sequence at positions 24 - 170 of β-lactamase (BLA) (N-terminal fragment) and the protein consisted of the amino acid sequence at positions 168 - 286 (C-terminal fragment), the binding of the given protein and the partner protein can be detected by the increase of the growth rate of the transformed cell. That is because β-lactamase has the activity of degrading ampicillin that prevents the growth of host cells of the transformed cell. Specific methods of growth assays utilizing the growth of the transformed cell include the method described in the following Example 4.

As for a detection kit of the present invention, it is not specially limited as long as it is provided with the fusion protein of the present invention which is a protein wherein a given enzyme activity exhibited by a given protein and/or a partner protein changes when the given protein and the partner protein are bound to each other compared to when they are not bound, or the transformed cell of the present invention; and a chromogenic substrate or a fluorogenic substrate that can be detected due to the change of the enzyme activity. However, an appropriate solvent may be further provided to suspend a sample when it is a solid.

The present invention will be explained more specifically by referring to the following Examples, but the technical scope of the present invention will not be limited to these exemplications. Referring to the outline of the following Examples, a vector expressing the fusion protein of the present invention was produced in Examples 1 to 2, and the growth assay of a transformant that expresses the fusion protein of the present invention was performed in Example 6. As a control experiment, a vector expressing the split mutant of the fusion protein of the present invention (the fusion protein of the present invention lacking the linker) was produced in Example 3, and the growth assay of the transformant expressing the split mutant was performed in Example 7. As a preparation for another comparative experiment, a vector expressing an antibody molecule deficient-mutant of the fusion protein of the present invention (the fusion protein lacking its antibody molecule in the fusion protein of the present invention) was produced in Example 4. Examples 5, 8, and 9 were performed to confirm that the results of the above-mentioned growth assays reflect the activity complementation of cpBLA. Further, in Examples 10 and 11, it was confirmed that the increase in the BLA activity of the fusion protein of the present invention in the presence of an antigen was remarkable compared with its split mutant or its antibody molecule deficient-mutant. Further, in Example 12, general versatility of the fusion protein of the present invention was confirmed by replacing the antibody molecule in the above-mentioned fusion protein of the present invention with another antibody molecule, and confirming that the BLA activity increases depending on the antigen in the presence of the antigen binding to the antibody molecule.

### Example 1

### [Preparation of circularly permutated β-lactamase (cpBLA) gene]

A DNA fragment (SEQ ID NO: 6) encoding an amino acid sequence at positions 24 - 170 of the wild-type BLA protein (SEQ ID NO: 5) and a DNA fragment (SEQ ID NO: 8) encoding an amino acid sequence at positions 168 - 286 (SEQ ID NO: 7) were generated by PCR using a wild-type β-lactamase (wild-type BLA) DNA sequence as a template. Specifically, the preparation was made by the following method.

First, the following four kinds of primers were arranged and used for the above-mentioned PCR reaction.
BLA24rev (SEQ ID NO: 9: CATTGGGACAAGAGCCACCCAGAAACGCTGGTGAAA)
BLA170for (SEQ ID NO: 10: GGCGATATCGGCTTCATTCAGCTCCGGTTC)
BLA168rev (SEQ ID NO: 11: GGCGATATCAATGAAGCCATACCAAAC)
BLA286for (SEQ ID NO: 12: TGGGTGGCTCTTGTCCCAATGCTTAATCAGTGA)
A linker sequence (a nucleotide sequence at positions 7 - 15 of SEQ ID NOs: 9 and 12) was arranged at the 5'-terminal of BLA24rev and BLA286for, and a EcoRV recognition sequence (a nucleotide sequence at positions 4 - 9^{th} position of SEQ ID NOs: 10 and 11) was arranged at the 5'-terminal of BLA170for and BLA168rev as a restriction enzyme cleavage site for inserting the produced DNA fragment.
According to Guntas et al. (Proc. Natl. Acad. Sci. USA, 102, 11224-11229 (2005)), these four kinds of primers were designed to generate gene fragments having new termini at positions 168 and 170 of BLA, located near the active site. For amplifying the DNA fragments encoding the above-mentioned amino acid sequence at positions 24 - 170, BLA24rev and BLA170for were used, and for amplifying the DNA fragment encoding the above-mentioned amino acid sequence at positions 168 - 286, BLA168rev and BLA286for were used.

Further, the above-mentioned PCR was carried out using pET20b (+) vector encoding the wild-type BLA gene (Merck KGaA) as a template, and Ex-Taq DNA polymerase (TAKARA-BIO INC.) as an enzyme. To be specific, 100 µl of PCR reaction mixture was prepared with two kinds of primers of 50 pmol each, 10 ng of pET20b(+), 0.2 mM (final concentration of reaction solution) of dNTPs, 10 µl of Ex-Taq Buffer, and 1 µl of 5 unit Ex-Taq, and the PCR was conducted at 94°C for 5 minutes, followed by repeating 25 cycles of 94°C for 30 seconds, 56°C for 30 seconds, and 72°C for 1 minute, thereafter incubating at 72°C for 10 minutes. The DNA fragment encoding the amino acid sequence at positions 24 - 170 and the DNA fragment encoding the amino acid sequence at positions 168 - 286 of the wild-type BLA protein were produced by the reaction. Subsequently, these two kinds of DNA fragments were subjected to electrophoresis with 1% of agarose gel containing a TAE buffer solution. And the DNA fragments were isolated by excising the gel slice containing the target band, and purified using Wizard (registered trademark) SV Gel and PCR Clean-Up System (Promega K.K.).

The original N-terminus (amino acid at position 24) and C-terminus (amino acid at position 286) of the generated two kinds of DNA fragments were linked via a DKS linker sequence (amino acid sequence: Asp-Lys-Ser; nucleotide sequence: gacaagagc) by an overlap extension PCR, and a PCR-generated fragment (circularly permutated gene), having position 168 and position 170 as a new N-terminal and a new C-terminal respectively, was created (construct at the upper left part of Fig.2; cpBLA). In further detail, the preparation was made by the following method.
100 µl of the reaction mixture was prepared with two kinds of PCR products of about 100 ng each (a DNA fragment encoding the amino acid sequence at positions 24 - 170 and a DNA fragment encoding the amino acid sequence at positions 168 - 286 of a wild-type BLA protein), 0.2 mM (final concentration of reaction solution) of dNTPs, 10 µl of Ex-Taq Buffer, and 5unit Ex-Taq, and the PCR was conducted at 94°C for 5 minutes, followed by repeating 10 cycles of 94°C for 30 seconds, 58°C for 30 seconds, and 72°C for 1 minute, thereafter incubating at 72°C for 10 minutes. Thus, the desired circularly permutated BLA gene was produced and named cpBLA (amino acid sequence: SEQ ID NO: 13; nucleotide sequence: SEQ ID NO: 14).

After the PCR, the reactionmixture was added with two kinds of primers (BLA168rev and BLA170for) of 50 pmol each and 2.5 unit Ex-Taq in order to amplify only the DNA of cpBLA. The PCR was conducted at 94°C for 5 minutes, followed by 25 cycles of 94°C for 30 seconds, 56°C for 30 seconds, and 72°C for 1 minute, incubating at 72°C for 10 minutes. The obtained reaction mixture was subjected to agarose gel electrophoresis, thereby confirming that the DNA fragment with expected size was amplified. Further, the gel containing the band of the expected size was excised and purified.

The purified DNA fragment was inserted into pCR4-TOPO (Invitrogen Inc.) and the nucleotide sequence of the construct was confirmed. Further detailed procedures are described as follows.
TOPO TA Cloning Kit for Sequencing (Invitrogen Inc.) is used. The kit contains pCR4-TOPO vector, and is appropriate for cloning and sequencing Taq polymerase-amplified PCR product with 3'-dA protruded terminus. A mixture was prepared with 1 µl of the pCR4-TOPO vector, equimolar amount of the aforementioned purified DNA fragment, and 1 µl of a salt solution, and placed at a room temperature for 30 minutes. The E. coli XL10-Gold strain (Tet^{r}, Δ(mcrA) 183, Δ(mcrCB-hsdSMR-mrr) 173, endA1, supE44, thi-1, recA1, gyrA96, relA, lac, The, [F', proAB, laclqZΔM15, Tn10 (Tet^{r}), Tn5(Kan^{r}), Amy]) was thawed on ice and added with the ligation mixture. The cells were placed on ice for 30 minutes, followed by heat shock step at 42°C for 45 seconds. Then the cells were added with 20 µl of an SOC culture medium (20 g of bacto tryptone, 5 g of bacto yeast extract, 0.5 g of NaCl, 0.2 ml of 5N NaOH, 20 ml of 1 M glucose, 10 ml of 1 M MgCl₂, 10 ml of 1 M MgSO₄ per L), and cured at 37°C for 30 minutes. Thereafter, the transformed cells were cultured at 37°C overnight on an LB agar medium plate (10 g of bacto tryptone, 5 g of bacto yeast extract, 5 g of NaCl, 0.2 ml of 5N NaOH, 15 g of agar per L) with 100 µg/ml ampicillin, 1% glucose, 10 µl of 0.4 M IPTG, and 200 µl of 10 mg/ml X gal. Next day, among some of blue and white colonies, white colonies were picked with a toothpick, and placed into colony PCR using Taq polymerase (TAKARA-BIO INC.) and two primers (T3 and T7). The PCR product was subjected to agarose gel electrophoresis, and only colonies which gave the band with the expected size were inoculated and cultured at 37°C overnight in a sterilized LB liquid medium (10 g of bacto tryptone, 5 g of bacto yeast extract, 5 g of NaCl, 0.2 ml of 5 N NaOH per L) containing 100 µg/ml ampicillin. Plasmids were extracted from the cultured cells by using Wizard (registered trademark) plus SV Minipreps (Promega K.K.), and the sequence of circularly permutated BLA (cpBLA) was confirmed using an ABI3100 DNA sequencer. The above-mentioned plasmid containing cpBLA was named cpBLA/TOPO.

### Example 2

### [Construction of the antibody-circularly permutated BLA fusion protein expression vector]

The variable domain of the antibody recognizing the neonicotinoid agrichemical imidacloprid (ICP) (a monoclonal antibody produced of a hybridoma deposited as 33C3-1-1: FERMP-17094: Japanese Laid-Open Patent Application No.2000-191698) was inserted into a phagemid vector pIT2 (Nature Biotechnol., 18, 989-994 (2000)). A phage displaying a single-chain antibody (scFv) was prepared according to a common method, thereby confirming its antigen binding ability (ScFv (ICP)/pIT2). The antibody gene was generated from the scFv (ICP)/pIT2, and transferred to a plasmid vector for overexpressing proteins, thereby producing scFv (ICP)/pET26. The detailed construction was made as follows.

The antibody gene contained in the scFv (ICP)/pIT2 was inserted into plasmid pET26b (+) (Merck KGaA), which allows expression and secretion of the target protein into periplasm, and also encodes kanamycin resistance. For restriction enzyme reaction, 50 µl of reaction mixture containing 1 µg each of scFv (ICP)/pIT2 and pET26b (+), 10 unit NcoI (New England Biolabs, Inc.), 10 unit NotI (New England Biolabs, Inc.), 5 µl of 10×NEB Buffer 3 (New England Biolabs, Inc.), 5 µl of 0.1% BSA (New England Biolabs, Inc.), and sterilized water was prepared, incubated at 37°C for 16 hours. The restriction enzymes-digested products were excised and purified following agarose gel electrophoresis. Ligation reaction was carried out at 16°C for 30 hours using 3 µl of the gel-purified scFv (ICP) gene fragment, 2 µl of the gel-purified vector, and 5 µl of Ligation High solution (TOYOBO CO., LTD.). Then, 5 µl of this reaction mixture was used for transformation of 100 µl E. coli TOP10 competent cells (F-mcrA, Δ(mrr-hsdRMS-mcrBC), φ802acZΔM15, ΔlacX74, deoR, recA1, araD139, Δ(ara, leu) 7697galU, galK, rpsL(Str^{R})endA1, nupG), and the cells were cultured at 37°C overnight on an LB agar medium plate containing 50 µg/ml of kanamycin. Next day, some colonies were picked up with a toothpick, and the colony PCR was performed using Taq DNA polymerase (TAKARA BIO, INC.) and two primers (T7 promoter and T7 terminator). The PCR product was subjected to agarose gel electrophoresis, and the colonies containing bands with expected size were inoculated and cultured in 4 ml of an LB liquid medium containing 50 µg/ml of kanamycin at 37°C overnight. ScFv (ICP)/pET26 was extracted from the cultured cells.

PCR was carried out using QuikChange mutagenesis kit (Stratagene Corporation: registered trademark), to introduce DraI site as a restriction enzyme site for integrating the DNA fragment encoding cpBLA, into a linker region of scFv (ICP)/pET26(construct at the upper right part of Fig.2). By digesting at this DraI site, scFv (ICP)/pET26 becomes a linear DNA having blunt ends and can ligate with EcoRV-digested cpBLA fragment also having blunt ends. The PCR for introducing the DraI site was performed as follows.
50 µl of the reaction mixture was prepared with the later mentioned two kinds of primers, 15 pmol each, 1 ng of scFv (ICP)/pET26, 0.2 mM (final concentration of the reaction solution) of dNTPs, 2.5 unit PfuUltra High-Fidelity DNA Polymerase (Stratagene Corporation), and 5 µl of 10 × PfuUltra reaction Buffer (Stratagene Corporation), and the reaction was conducted at 95°C for 1 minute, followed by 18 cycles of 95°C for 30 seconds, 55°C for 30 seconds, and 68°C for 15 minutes. The sequences of the used primers are as follows. Dralrev (SEQ ID NO: 15: GGTGGAGGCGGTTCAGGCTTTAAAGGCAGTGGCGGTGGCGGG), Dralfor (SEQ ID NO: 16: CCCGCCACCGCCACTGCCTTTAAAGCCTGAACCGCCTCCACC) (nucleotide sequences at positions 19 - 24 of SEQ ID NOs: 15 and 16 are a DraI cleavage site, respectively)

After the PCR, DpnI (1 µl) was added to the reaction mixture, and incubated at 37°C for 1 hour to degradate methylated template DNA. Then, 5 µl thereof, 100 µl of the mixture was used for transformation of the E. coli TOP10 competent cells. The cells were cultured at 37°C overnight on an LB agar medium containing 50 µl/ml of kanamycin. Next day, some of the generated colonies were picked by a toothpick, and were inoculated and cultured in 4 ml of an LB agar medium containing 50 µl/ml of kanamycin at 37°C overnight. After extracting a plasmid from the cultured cells, the sequence was determined using an ABI3100DNA sequencer, and the DraI site was confirmed to be introduced at the desired position.

Subsequently, scFv(ICP)/pET26 introduced with a DraI site was cleaved using the DraI to prepare scFv (ICP)/pET26. EcoRV treated cpBLA was inserted into the scFv (ICP) /pET26, and an antibody-circularly permutated enzyme expression vector-V_{H}-cpBLA-V_{L}/pET26 (the construct at lower middle part of Fig. 2) was produced. The detailed procedures are explained in the following.
50 µl of reaction mixture containing 1 µg of scFv (ICP)/pET26, 20 unit DraI (New England Biolabs Inc.), 10 unit Alkaline Phosphotase, Calf Intestinal (New England Biolabs Inc.), and 10 × NEB Buffer4 (New England Biolabs Inc.) and sterilized water, was incubated at 37°C for 16 hours. In order to prevent self-ligation of the vector, dephosphorylation of the termini was performed with alkaline phosphatase along with the restriction enzyme treatment. Further, 50 µl of reaction mixture containing 1 µg of pCR4-TOPO vector inserted with a cpBLA fragment, 20 unit EcoRV (New England Biolabs Inc.), 10 × NEB Buffer3, and 0.1% of BSA, and sterilized water was prepared, and incubated at 37°C for 16 hours. The restriction enzymes-digested products were excised and purified following agarose gel electrophoresis. Ligation reaction was carried out at 16°C for 30 hours using 3 µl of scFv (ICP)/pET26 vector, 3 µl of the gel-purified cpBLA fragment, and 6 µl of Ligation High solution. Then, 5 µl of this reaction mixture was used for transformation of 100 µl of the E. coli TOP10 strain, and the cells were cultured at 37°C overnight on an LB agar medium plate containing 50 µg/ml of kanamycin. Next day, some colonies were picked with a toothpick, and the colony PCR was performed using Taq DNA polymerase and two primers (T7 promoter and BLA170for). The PCR product was subjected to agarose gel electrophoresis, and the colonies containing bands with expected size were inoculated and cultured in 4 ml of an LB liquid culture medium containing 50 µg/ml of kanamycin at 37°C overnight. V_{H}-cpBLA-V_{L}/pET26 (antibody-circularly permutated BLA fusion protein expression vector) was extracted from the cultured cells.

### Example 3

### [Construction of antibody-split-circularly permutated BLA fusion protein expression vector]

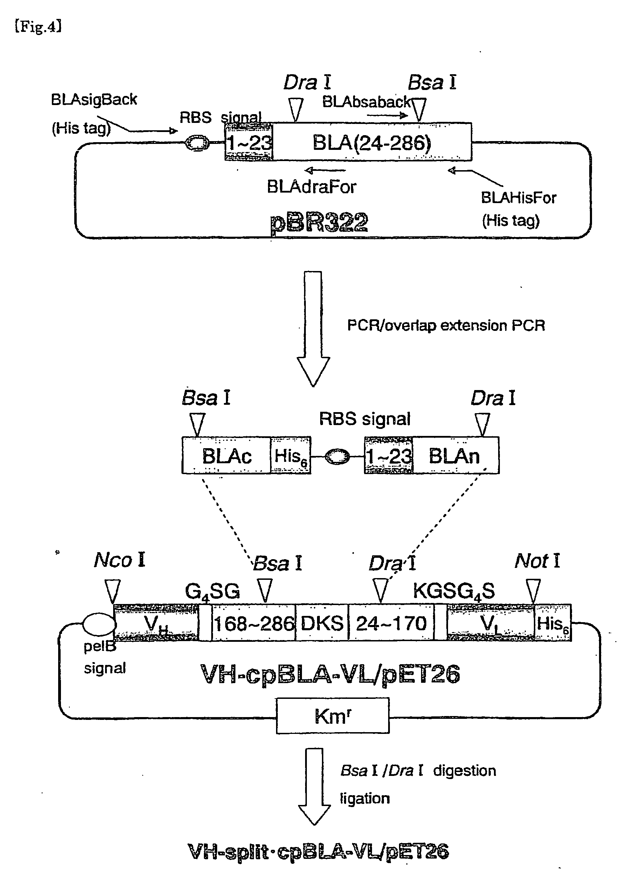

In order to study the significance of a linker peptide in the fusion protein of the present invention, V_{H}-split-cpBLA-V_{L}/pET26 (antibody-split-circularly permutated BLA fusion protein expression vector) was constructed by replacing a sequence encoding a linker peptide in V_{H}-cpBLA-V_{L} with a sequence encoding stop codon and RBS. The construction of the vector is shown in Fig.3. The V_{H}-split-cpBLA-V_{L}/pET26 is, as shown in Fig. 3, a vector that expresses V_{H}-BLAc (a fusion protein of a V_{H} domain polypeptide and an amino acid sequence at positions 168 - 286 of a wild-type BLA protein) and BLAn-V_{L} (a fusion protein of a V_{L} domain polypeptide and an amino acid sequence at positions 24 - 170 of a wild-type BLA protein). V_{H}-split-cpBLA-V_{L}/pET26 was constructed by the following procedures in accordance with the outline shown in Fig.4.

First, pBR322 with the schematic structure as shown in Fig.4 was prepared. PCR was conducted using pBR322 as a template, two kinds of primers, BLAsigBack (SEQ ID NO: 17: TCACCATCACTAAGAAGGAGATATCATATGAGTATTCAACATTTCC) (the 1^{st} to 18^{th} nucleotide from the 5'-terminal is the overlapped domain with BLAHisFor) and BLAdraFor (SEQ ID NO: 18: CGGCGACCGAGTTGCTCTTG). The PCR generated an amplified-fragment (BLA-DraI fragment) containing from an ampicillin resistance gene through a DraI restriction site located in the anterior half of the BLA via a signal sequence of pBR 322. Also, PCR reaction was conducted using pBR322, two primers BLAbsaBack (SEQ ID NO: 19: ATGGAGGCGGATAAAGTTGC) and BLAHisFor (SEQ ID NO: 20: CCTTCTTAGTGATGGTGATGATGATGCCAATGCTTAATCAGTGAGGC) (the 1^{st} to 18^{th} nucleotide from the 5'-terminal is the overlapped domain with BLAsigBack). The PCR generated an amplified-fragment (BLA-BsalI fragment) containing from a BsaI site in the latter half of BLA to the 296^{th} position of BLA protein. In addition, the 5'-terminal of BLAHisFor contains the complementary strand of the sequence encoding His tag, by which the His tag for purification is added at the C-terminus of V_{H}-BLAc. PCR for amplifying a BLA-DraI fragment or a BLA-BsaI fragment was performed as follows.

100 µl of the reaction mixture was prepared with two kinds of primers (BLAsigBack and BLAdraFor for amplifying the BLA-DraI fragment; BLAbsaBack and BLAHisFor for amplifying the BLA-BsaI fragment) of 50 pmol each, 10 ng of pBR322, 0.2 mM (final concentration of reaction solution) of dNTPs, 10 µl of 10xEx-Taq buffer, and 5 unit of Ex-Taq, and the PCR reaction was conducted at 95°C for 5 minutes, followed by 25 cycles of 95°C for 30 seconds, 50°C for 30 seconds, and 72°C for 30 seconds, thereafter incubating at 72°C for 5 minutes. The BLA-DraI fragment and BLA-BsaI fragment generated by the PCR reaction were subjected to electrophoresis respectively with 1.5% of agarose gel containing a TAE buffer solution. The gel containing the band with the expected size was excised and purified.

Subsequently, by performing overlap-extension PCR for the purified BLA-DraI fragment and BLA-BsaI fragment, the BLA-DraI fragment and the BLA-BsaI fragment were bound to each other (see the construct in the middle of Fig. 4). To be specific, the overlap-extension PCR was performed as follows.
100 µl of the reaction mixture was prepared with the purified BLA-DraI fragment (about 100 ng) and BLA-BsaI fragment (about 100 ng), 0.2 mM (final concentration of reaction solution) of dNTPs, 10 ml of 10xEx-Taq buffer, and 5 unit of Ex-Taq. The PCR reaction was conducted at 95°C for 5 minutes, followed by 15 cycles of 95°C for 30 seconds, 55°C for 30 seconds, and 72°C for 45 seconds, thereafter incubating at 72°C for 5 minutes. Subsequently, to further increase the number of target DNA fragments, the obtained reaction mixture was added with BLAbsaBack and BLAdraFor primers (50 pmol each), and 2.5 unit of Ex-Taq. And the reaction was conducted by repeating 25 cycles of 95°C for 30 seconds, 55°C for 30 seconds, 72°C for 45 seconds, further incubating at 72°C for 5 minutes, thereby obtaining the reaction mixture. The obtained reaction mixture was subjected to agarose gel electrophoresis, thereby confirming that the DNA fragment with expected size was amplified. Further, the gel near the expected size was excised to purify the DNA fragment with expected size.

The obtained DNA fragment was inserted into pCR4-TOPO (Invitrogen Inc.) and its nucleotide sequence was confirmed. The detailed procedures are as follows.
TOPO TA Cloning Kit for Sequencing (Invitrogen Inc.), containing pCR4-TOPO vector, and being appropriate for cloning and sequencing Taq polymerase-amplified PCR product with 3'-dA protruded terminus. A mixture was prepared with 1 µl of the pCR4-TOPO vector, an equimolar amount of the above-mentioned DNA fragment, and a salt solution, and placed at a room temperature for 30 minutes. The E. coli XL10-Gold (Tet^{r}, Δ(mcrA) 183, Δ(mcrCB-hsdSMR-mrr) 173, endA1 supE44, thi-1, recA1, gyrA96, relA, lac, Hte, [F', proAB, laclqZΔM15, Tn10 (Tet^{r}), Tn5 (Kan^{r}), Amy] was thawed on ice and added with the ligation mixture. The cells were placed on ice for 30 minutes, followed by a heat shock step at 42°C for 45 seconds. Then the cells were added with 20 µl of an SOC medium (20 g of bacto tryptone, 5 g of bacto yeast extract, 0.5 g of NaCl, 0.2 ml of 5N NaOH, 20 ml of 1 M glucose, 10 ml of 1 M MgCl₂, and 10 ml of 1 M MgSO₄ per L), and cured at 37°C for 30 minutes. Thereafter, the transformed cells were cultured at 37°C overnight on an LB agar medium plate (10 g of bacto tryptone, 5 g of bacto yeast extract, 5 g of NaCl, 0.2 ml of 5N NaOH, and 15 g of agar per L) with 100 µg/ml ampicillin, 1% glucose, 10 µl of 0.4 M IPTG, and 200 µl of 10 mg/ml X-gal. Next day, among some of blue and white colonies, white colonies were picked with a toothpick. And colony PCR was carried out using the white colonies, Taq DNA polymerase (TAKARA-BIO INC.) and two primers (T3 and T7). The PCR product was subjected to agarose gel electrophoresis, and the colonies containing bands with expected size were inoculated and cultured at 37°C overnight in a sterilized LB liquid culture medium (10 g of bacto tryptone, 5 g of bacto yeast extract, 5 g of NaCl, and 0.2 ml of 5 N NaOH per L) with 100 µg/ml ampicillin. After extracting a plasmid from the cultured cells, the sequence of the aforementioned purified DNA fragment was determined using an ABI3100 DNA sequencer, and its sequence was confirmed to be the split·circularly permutated BLA (split-CpBLA) (see the construct in the middle of Fig. 4). The above-mentioned plasmid containing the split·cpBLA was named split·cpBLA/TOPO.

50 µl of a reaction mixture was prepared with 1 µg of split-cpBLA/TOPO, 10 unit DraI and 5 µl of NEBuffer2, and milliQ water, and incubated at 37°C for 6 hours. 5 unit of BsaI was added to the reaction mixture, and placed at 50°C overnight for restriction enzyme digestion. Similarly, the restriction enzyme digestion with DraI and BsaI was performed using 1 µg of V_{H}-cpBLA-V_{L}/pET26 instead of using 1 µg of split·cpBLA/TOPO. Each solution obtained from the restriction enzyme digestion was migrated in an agarose gel, 5 µl of purified DNA solution was added with 10 µl of Ligation high ver.2 (TOYOBO CO., LTD.), and ligation reaction was carried out at 16°C for 30 minutes. Then, 5 µl of this reaction mixture was used for transformation of 100 µl of the E. coli TOP10 strain, and the cells were cultured at 37°C overnight on an LB agar medium plate containing 50 µg/ml of kanamycin. Next day, after further culturing single colonies in 4 ml of LBK overnight, plasmid was extracted from the cultured cells, and the DNA sequence of the plasmid was confirmed. From several plasmids whose DNA sequence was confirmed, a plasmid (Fig.4) in which the sequence between BsaI site and DraI site in V_{H}-cpBLA-V_{L}/pET26 was replaced by the sequence between BsaI site·DraI site in the split and cpBLA was found out, and this plasmid was named as V_{H}-split·cpBLA-V_{L}/pET26 (Fig.3).

### Example 4

### [Construction of the circularly permutated BLA protein expression vector]

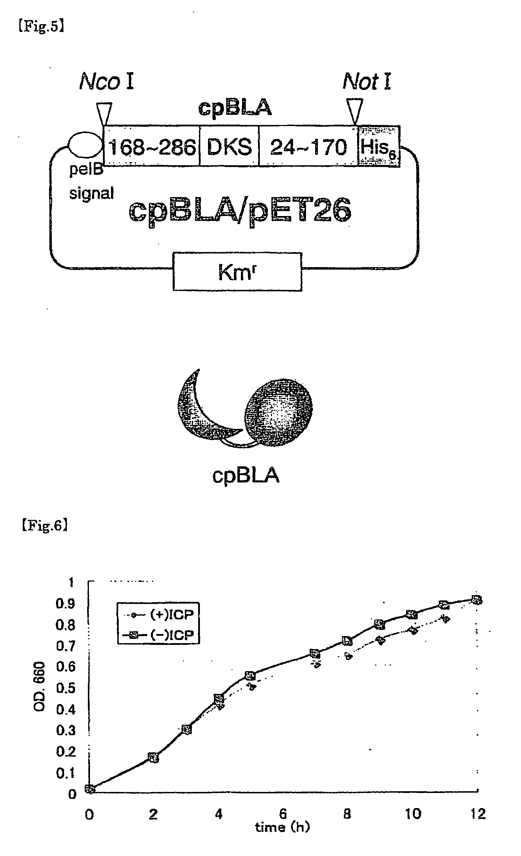

An expression vector containing only cpBLA without antibody gene (circularly permutated BLA protein expression vector) (Fig.5) was constructed as a control of V_{H}-cpBLA-V_{L}/pET26, one of the fusion proteins of the present invention. In further detail, the construction was made by the following procedures.

A cpBLA gene which was added with an NcoI site and an XhoI to the termini was amplified by PCR using cpBLA/TOPO constructed in Example 1 as a template, and was inserted into a pET26b (+). This PCR was performed as follows.
100 ml of the reaction mixture was prepared with 50 pmol of BLA168Ncorev (SEQ ID NO: 21: CATGCCATGGGCAATGAAGCCATACCAAAC) (the 5^{th} to 10^{th} nucleotide from the 5'-terminal is an NcoI site), 50 pmol of BLA170Xhofor (SEQ ID NO: 22: CCGCTCGAGGCTTCATTCAGCTCCGGTTC) (the 4^{th} to 9^{th} nucleotide from the 5'-terminal is an Xho site), 10 ng of V_{H}-cpBLA-V_{L}/TOPO (a plasmid integrating V_{H}-cpBLA-V_{L} into pCR4-TOPO), 0.2 mM (final concentration of reaction solution) of dNTPs, 10 µl of 10'Ex-Taq buffer, and 5 unit Ex-taq, and the PCR reaction was conducted at 94°C for 5 minutes, followed by 25 cycles of 94°C for 30 seconds, 65°C for 30 seconds, and 72°C for 1 minute, thereafter incubating at 72°C for 10 minutes, thereby obtaining the reaction mixture. The obtained reaction mixture was subjected to agarose gel electrophoresis for purification, and digested with NcoI and XhoI. The restriction enzymes-digested mixture was subjected to agarose gel electrophoresis for purification. The purified DNA fragment was also digested with NcoI and XhoI. After purification, the digested DNA fragment was inserted into pET26b (+) to make cpBLA/pET26 (Fig.5).

### Example 5

### [Toxicity test of ICP in E. coli]

First, in order to study that ICP has no toxicity or growth-promoting activity to E. coli as a conduction of the growth assay of the later mentioned Example 6, growth of the wild-type BL21 (DE3, pLysS) strain in the culture medium containing high concentration of ICP was studied by the following procedures.
The BL21 (DE3, pLysS) strain was cultured at 30°C overnight on an LB agar medium plate containing 37 µg/ml of chloramphenicol. The generated colonies were picked with a toothpick, inoculated and cultured in a 4 ml of the LB liquid culture medium containing 37 µg/ml of chloramphenicol at 27°C overnight. 40 µl of this pre-cultured medium was inoculated and cultured in a 4 ml of the liquid culture medium containing 37 µg/ml of chloramphenicol and 100 ng/ml of ICP at 27°C overnight, with measuring the time course of turbidity change using Mini photo 518R (TAITEC CORPORATION) at 660 nm. Further, the time course of change in turbidity was measured similarly using the same LB liquid culture medium except that it did not contain ICP. The results are shown in Fig.6. As observed from Fig.6, the E. coli BL21 (DE3, pLysS) strain showed similar growth curves regardless of the presence or absence of ICP. Therefore, it is considered that ICP has no toxicity or growth-promoting activity in E. coli.

### Example 6

### [Growth assay using V_{H}-cpBLA-V_{L} expressing strain]

In order to study whether the V_{H}-cpBLA-V_{L} (antibody-enzyme fusion protein) expressing strain can be used to detect the presence or absence of the target antigen, growth assay was carried out using ampicillin that can be the substrate of the BLA activity exhibited when the target antigen is bound to the antibody-enzyme fusion protein. Also, for the growth assay, ICP which is the target antigen of this antibody-enzyme fusion protein, and the thiacloprid (TCP) which is an analog of ICP with a lower cross-reactivity than ICP were used. In further detail, the assay was performed by the following procedures.

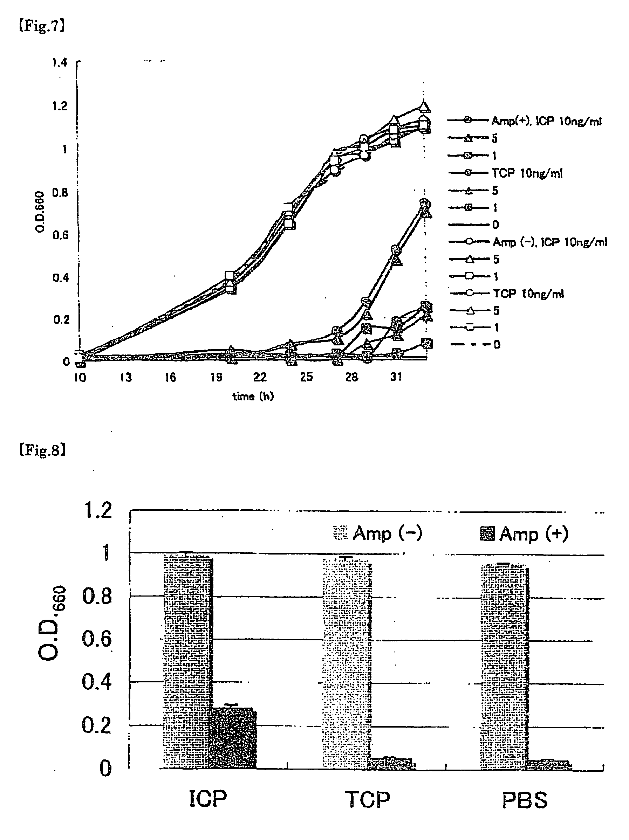

First, the strain expressing V_{H}-cpBLA-V_{L} (antibody-enzyme fusion protein) was prepared.
0.5 µg of V_{H}-cpBLA-V_{L}/pET26 was used for transformation of 100 µl of the BL21 (DE3, pLysS) strain (F-, ompT, hsdSB, (rB-, mB-), dcm, gal, λ(DE3), pLysS, Cmr), and the transformed cells were cultured on an LB agar medium plate containing 50 µg/ml of kanamycin and 37 µg/ml of chloramphenicol at 30°C overnight, to prepare an antibody-enzyme fusion protein expressing strain (V_{H}-cpBLA-V_{L}/pET26/BL21 strain). After the overnight culture, colonies generated on the above-mentioned LB agar medium plate were picked with a toothpick, inoculated and cultured in a 4 ml of the LB liquid culture medium plate containing 50 µg/ml of kanamycin and 37 µg/ml of chloramphenicol at 27°C overnight. 4 µl of this pre-cultured medium was inoculated and cultured in 4 ml of the LB liquid culture medium containing 50 µg/ml of kanamycin and 37 µg/ml of chloramphenicol, 1 mM of IPTG, 0 or 100 µg/ml ampicillin, 0 to 10 ng/ml of ICP or TCP at 27°C for 33 hours, with measuring the time course of turbidity change using Mini photo 518R at 660 nm. The result is shown in Fig. 7.

As observed from Fig.7, in the 100 µg/ml amplicillin-containing LB culture medium, a dose-dependent growth caused by ICP was observed at 5 to 10 ng/ml from about 24 hours after the initiation of measurement. Also, a dose-dependent growth caused by ICP at a low concentration of 1 ng/ml was observed from about 30 hours after the initiation of measurement. In contrast, growth caused by TCP was observed from about 30 hours which was later than when the ICP of the same concentration was added. And when 10 ng/ml of TCP was added, a significant growth was observed similarly as when 1 ng/ml of the ICP was added. On the other hand, in the culture medium without ampicillin, similar growth was observed earlier than the ampicillin-containing culture medium regardless of the kinds of antigens or concentration thereof (Fig. 7). It is worth noting that the criterion values of ICP allowed to be contained in the agricultural products in Japan are 20 to 5000 ng/ml (http://m5.ws001.squarestart.ne.jp/zaidan/agrdtl.php?a_inq= 8800), and it can be said that the present result shows that the protein of the present invention has a satisfactory sensitivity for the target antigen.

### Example 7

### [Growth assay using V_{H}-split·cpBLA-V_{L} expressing strain and the like]

By applying the assay of the above-mentioned Example 6, VH-split·cpBLA-V_{L}/pET26 was used instead of V_{H}-cpBLA-V_{L}/pET26, and the E. coli C43 (DE3) strain (Lucigen Corporation) suitable for overexpression of a highly toxic protein was used instead of the BL21 (DE3, pLysS) strain, to investigate the expression in the strains. To be specific, 0.5 µg of V_{H}-split·cpBLA-V_{L}/pET26 was used for transformation for the C43 (DE3) strain, and the transformed cells were cultured on a LB agar medium plate containing 50 µg/ml of kanamycin at 30°C overnight, and the V_{H}-split·cpBLA-V_{L} expressing strain (V_{H}-split·cpBLA-V_{L}/pET26/C43 strain) was prepared. After overnight culture, the colonies generated on the above-mentioned LB agar medium plate were picked with a toothpick, inoculated and cultured in a 4 ml of the LB liquid medium containing 50 µl of kanamycin (LBK) at 27°C overnight. 10 µl of this pre-cultured medium was inoculated in 1 ml of the LBK liquid medium containing 1 mM of IPTG, 0 or 10 µg/ml of ampicillin, 10 ng/ml of ICP or TCP. After the cells were cultured at 27°C for 45 hours, the turbidity was measured using Mini photo 518R at 660 nm. The result is shown in Fig. 8. As observed from Fig. 8, whereas no difference in growth was observed in the absence of ampicillin, the ICP dependent-growth was finally observed in the presence of 10 µg/ml ampicillin which is an amount smaller than in Example 6. However, when 100 µg/ml ampicillin was added, or when less than 100 µg/ml ICP or TCP was added, no significant growth could be confirmed. Further, since the growth was not observed using the BL (DE3, pLysS) strain, it was considered that V_{H}-split·cpBLA-V_{L} caused a lower antigen-dependent enzyme activity compared with V_{H}-cpBLA-V_{L}, and resulted in a low antigen detecting sensitivity.

In addition, a similar growth assay was performed using the cpBLA expressing strain (the cpBLA/pET26/BL21 strain or the cpBLA/pET26/C43 strain), and antigen-dependent growth was not observed in any of the cpBLA expressing strain. When ampicillin was not added, growth was observed regardless of the presence or absence of the antigen, while when ampicillin was added, growth was not observed in any of the samples.

### Example 8

### [Confirmation on the expression of the antibody-enzyme fusion protein by Western blotting]

Since the growth assay of the Example 6 could not deny the possibility that the difference in the growth speed was caused by the change in the V_{H}-cpBLA-V_{L} (antibody-enzyme fusion protein) expression level due to the presence or absence or the difference of the antigens in the culture medium, the expression level of the antibody-enzyme fusion protein in the antibody-enzyme fusion protein expressing strain was confirmed by the Western blot using an anti-BLA antibody. In further detail, the confirmation was made by the following procedures.

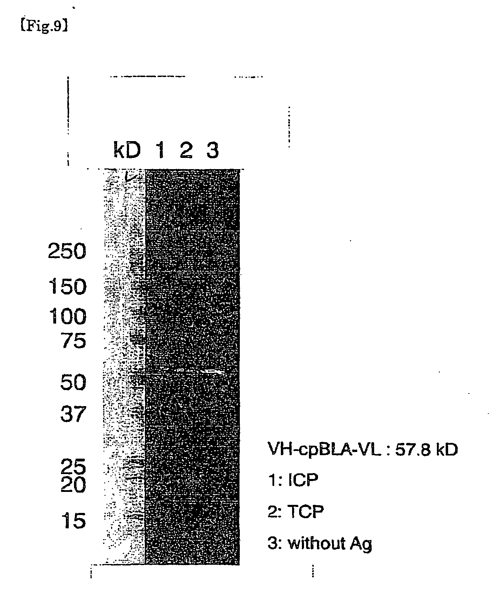

The antibody-enzyme fusion protein expressing strain (V_{H}-cpBLA-V_{L}/pET26/BL21 strain) was inoculated and cultured in 4 ml of the LB liquid medium containing 50 µg/ml of kanamycin and 37 µg/ml of chloramphenicol at 27°C overnight. A part (4 µl) of this pre-cultured medium was inoculated and cultured in 4 ml of the LB liquid medium containing 50 µg/ml of kanamycin, 37 µg/ml of chloramphenicol, 1 mM of IPTG, and 10 ng/ml of ICP or TCP at 27°C overnight. It was cultured overnight by the same method except that neither ICP nor TCP was contained. After the O.D.₆₆₀ of the culture reached around 0.6, a part (100 µl) of the culture was centrifuged at 15000 rpm for 5 minutes. Then, the cultured cells were collected and subjected to SDS-PAGE. The protein band in the migrated gel was transferred on a nitrocellulose membrane (Bio-Rad Laboratories, Inc), and the membrane was blocked with Immunoblock (Dainippon Sumitomo Pharma Co., Ltd) at 4°C. This nitrocellulose membrane was washed 3 times for 5 minutes with PBS-T (10 mM phosphate, 145 mM NaCl, 5 mM KCl, pH 7.2, added with 0.1% of Tween-20), and incubated with 5% of Immunoblock solution containing an anti-rabbit BLA polyclonal antibody at 1/5000 dilution (Chemicon Co., Ltd) at room temperature for 1 hour. After the incubation, the nitrocellulose membrane was washed 3 times with PBS-T, and incubated in 5% of the Immunoblock solution containing 1 mg/ml of HRP-Goat anti Rabbit at 1/5000 dilution at room temperature for 1 hour. The nitrocellulose membrane after incubation was washed 3 times with PBS-T. The HRP activity of the membrane was detected by SuperSignal WestPico chemiluminescent substrate (Pierce Biotechnology Inc.). The results are shown in Fig.9. Lane 1 shows a bacteria sample cultured in the presence of ICP, Lane 2 shows a bacteria sample cultured in the presence of TCP, and Lane 3 shows a bacteria sample cultured in the medium without ICP or TCP andtigen (Ag).

As observed from Fig. 9, it was demonstrated that a similar amount of the protein (V_{H}-cpBLA-V_{L}) was expressed near 58 kD (Fig.9) in all samples regardless of the kinds of the antigens. Therefore, it is considered that the increase in the growth rate due to the addition of an antigen (particularly ICP) is not caused by the increase of the expression level of the antibody-enzyme fusion protein (V_{H}-cpBLA-V_{L}), but by the increase of the enzyme activity induced by the structural stabilization of the antibody-enzyme fusion protein by the antigen binding.

### Example 9

### [Confirmation of the antibody-enzyme fusion protein expression by SDS-PAGE and Western blotting]

The antibody-enzyme fusion protein was partially purified from the expressing strain in order to confirm whether the antibody-enzyme fusion protein (V_{H}-cpBLA-V_{L}) is actually expressed in the antibody-enzyme fusion protein expressing strain (V_{H}-cpBLA-V_{L}/pET26/BL21 strain). In further detail, the partial purification was performed by the following procedures.

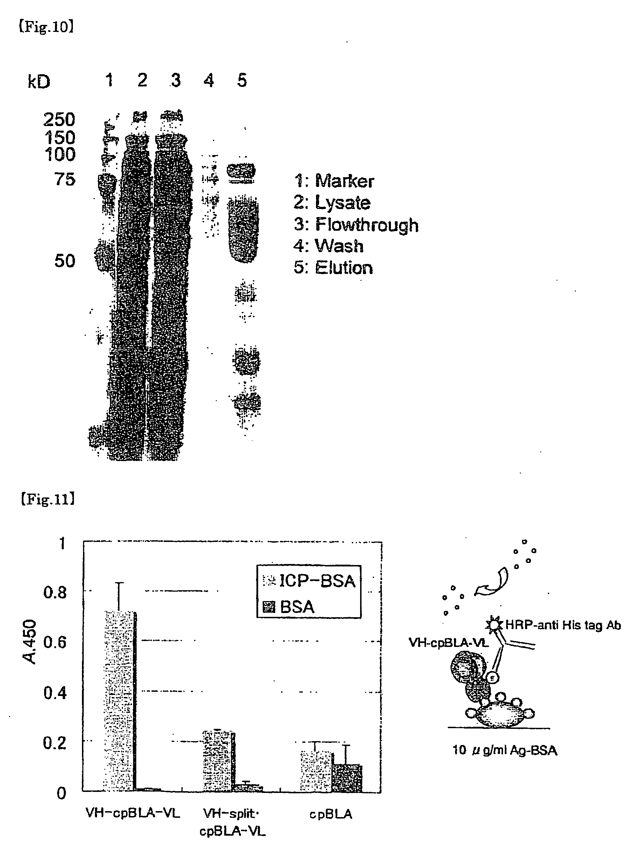

The antibody-enzyme fusion protein expressing strain (V_{H}-cpBLA-V_{L}/pET26/BL21 strain) was inoculated and cultured in 4 ml of the LB liquid medium containing 50 µg/ml of kanamycin and 37 µg/ml of chloramphenicol (LBKC medium) at 27°C overnight. This culture solution was inoculated in 250 ml of the LBKC liquid medium. When O.D.₆₀₀ of the culture reached around 0.6, 250 µl of 1 M of IPTG was added to induce the expression. After further incubation at 27°C for 16 hours, the culture solution was centrifuged at 6000 g for 10 minutes at 4°C. The supernatant was collected and added with 107. 5 g of ammonium sulfate. Then, the supernatant was placed at 4°C overnight, and the soluble protein was precipitated. The solution was centrifuged at 10000 g for 20 minutes at 4°C, and the supernatant was discarded. The precipitation was suspended in 10 ml of TALON Buffer (50 mM phosphate, 300 mM NaCl, pH 7.0). TALON metal affinity column (200 µl)(Clontech Laboratories, Inc.) was added to the suspension, and the protein was purified by a batch/gravity-flow procedure according to the instructions. Proteins were eluted with elution buffer (125 mM Imidazole, 50 mM phosphate, 300 mM NaCl, pH 7.0). With this concentrated protein, SDS-PAGE was performed. The results are shown in Fig. 10.

As observed in Fig.10, the band corresponding to the molecular weight of the antibody-enzyme fusion protein (V_{H}-cpBLA-V_{L}) being the target protein was confirmed near 58kD of Lane 5.

Further, the V_{H}-cpBLA-V_{L}/pET26/BL21 strain, V_{H}-split·cpBLA-V_{L}/pET26/BL21 strain, and cpBLA/pET26/BL21 strain obtained by transforming the BL21 (DE3, pLysS) strain with vector of V_{H}-cpBLA-V_{L}/pET26, V_{H}-split·cpBLA-V_{L}/pET26, and cpBLA/pET26, respectively, were inoculated and cultured in 4 ml of the aforementioned LBKC medium respectively at 27°C overnight. This culture solution was inoculated in 250 ml of the LBKC liquid medium. When O.D.₆₀₀ of the culture reached around 0.6, 250 µl of 1 M of IPTG was added to induce the expression. Afterfurtherincubation at 27°C for 16 hours, the culture solution was centrifuged at 6000 g for 10 minutes at 4°C. The supernatant was collected and added with 107.5 g of ammonium sulfate. Then, the supernatant was placed at 4°C overnight, and the soluble protein was precipitated. The solution was centrifuged at 10000 g for 20 minutes at 4°C, and the supernatant was discarded. The precipitation was suspended in 10 ml of TALON Buffer (50 mM phosphate, 300 mM NaCl, pH 7.0). TALON metal affinity column (200 µl) was added to the suspension, and the protein was purified by a batch/gravity-flow procedure according to the instructions. Proteins were eluted with elution buffer (125 mM Imidazole, 50 mM phosphate, 300 mM NaCl, pH 7.0). After performing SDS-PAGE using 10 µl of this concentrated protein solution, Western blotting was performed by the same method as in Example 8. From the results of Western blotting, bands were observed near the theoretical size of the target protein, respectively. Specifically, a band considered to be V_{H}-cpBLA-V_{L} was observed near 58kD from the V_{H}-cpBLA-V_{L}/pET26/BL21 strain. A band considered to be V_{H}-BLA_{C} was observed near 28kD and a band considered to be BLAn-V_{L} was observed near 31kD from the V_{H}-split·cpBLA-V_{L}/pET26/BL21 strain, and a band considered to be cpBLA was observed near 30kD from the cpBLA-V_{L}/pET26/BL21 strain.

### Example 10

### [Confirmation on the antigen binding ability of the antibody-enzyme fusion protein (V_{H}-cpBLA-V_{L}) and the like]

The following ELISA assay was performed to confirm whether the antibody-enzyme fusion protein (V_{H}-cpBLA-V_{L}) actually has a binding activity to the antigen (ICP).
PBS solution containing 10 µg/ml of ICP-BSA or BSA was dispensed into Falcon 3912 microplates by 100 µl each, and left at 4°C for 16 hours. After discarding the solution from the microplates, 200 µl of PBS including 25% of Immunoblock was added and left at a room temperature for 2 hours for blocking. Next, after washing the microplates with PBS-T, 50 µl/ml of the purified antibody-enzyme fusion protein (V_{H}-CpBLA-V_{L}) prepared in the above-mentioned Example 9 and 100 µl of PBS containing 5% Immunoblock were added, and left at a room temperature for 90 minutes. In order to detect the antibody-enzyme fusion protein (V_{H}-cpBLA-V_{L}) bound to the solid-phase by the above preparation, the microplates were washed with PBS-T, and added with HRP labeled Penta-His (QIAGEN K.K., Tokyo) diluted in 1/2000 in PBS containing 5% Immunoblock. After incubation at a room temperature for 1 hour, the microplates were washed 3 times with PBS-T, and added with 100 µl per well of the enzyme reaction solution (50 ml ELISA buffer, 500 µl TMBZ (in DMSO), 10 µl H₂O₂) to start the reaction. After incubation for 5 minutes in a dark place, the reaction was stopped by adding 50 µl of 3.2 N of H₂SO₄, and the absorbance was measured with a platereader (450 nm).

Also, the above-mentioned ELISA was applied for V_{H}-split·cpBLA-V_{L}(V_{H}-BLAc and BLAn-V_{L}) or cpBLA instead of using V_{H}-cpBLA-V_{L}, and the absorbance was measured similarly. The result is shown in Fig.11. As observed from Fig.11, compared with the cpBLA case, a significant ICP-BSA specific binding was confirmed using both V_{H}-cpBLA-V_{L} and V_{H}-split·cpBLA-V_{L}. Also, a prominent ICP-BSA specific binding ability was confirmed using V_{H}-cpBLA-V_{L}, compared with using the V_{H}-split·cpBLA-V_{L}. Further, considering the presence of His tag in both V_{H}-BLAc and BLAn-V_{L} in V_{H}-split·cpBLA-V_{L}, the level of the signal of V_{H}-cpBLA-V_{L} compared with that of V_{H}-split·cpBLA-V_{L} is more than what is shown in Fig.11. Moreover, since a similarly low signal is shown in cpBLA regardless of the presence or absence of ICP (antigen), it is possible that the conformation of BLA is changed and bound in a non-specific manner.

### Example 11

### [Change in the activity by the antigen binding of the antibody-enzyme fusion protein (V_{H}-cpBLA-V_{L})]

In order to confirm whether the antibody-enzyme fusion protein (V_{H}-cpBLA-V_{L}) binding to the antigen resulted in a change (increase) of the enzyme activity, the following experiment was performed using a chromogenic substrate Nitrocefin (Merck KGaA) or fluorogenic substrate Fluorocillin (Invitrogen Inc.), and concentrated V_{H}-cpBLA-V_{L} prepared in Example 9.

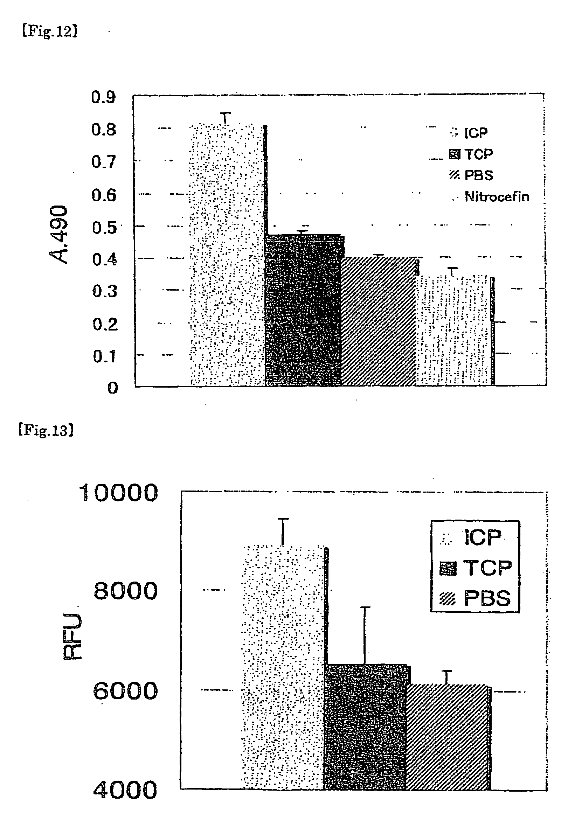

First, 5 µl of the concentrated V_{H}-cpBLA-V_{L} protein solution prepared in Example 9, 5 µl of 10 µg/ml ICP or TCP, and 90 µl of PBS containing 1 mM of Nitrocefin were mixed, and left in a dark place at a room temperature for 18 hours. Then, the absorbance at 490 nm of the reaction solution was measured to investigate the change in the β-lactamase activity. Further, absorbance at 490 nm was also measured when PBS was used instead of ICP or TCP (the item "PBS" in Fig.12), or when V_{H}-cpBLA-V_{L} protein solution was not added (the item "Nitrocefin" in Fig. 12). The results are shown in Fig. 12. As observed from Fig. 12, a significant signal increase in the sample added with ICP was observed compared with samples without added ICP ("TCP", "PBS", and "Nitrocefin"). In other words, the ICP-dependent increase of β-lactamase activity was observed. In addition, in a sample added with TCP, a slight significant signal increase (n = 2) was observed compared with samples without ICP or TCP ("PBS"). From these results, it is considered that the enzyme activity of the fusion protein is activated by adding the antigen.

Next, a similar experiment was performed using a fluorogenic substrate Fluorocillin instead of the chromogenic substrate Nitrocefin. Specifically, 5 µl of concentrated V_{H}-cpBLA-V_{L} protein solution prepared in Example 9, and 5 µl of 10 µg/ml ICP or TCP were mixed, and incubated at 4°C for 30 minutes. Then, 90 µl of PBS containing 10 µM Fluorocillin was added, and incubated at a room temperature for 30 minutes. The fluorescence at 535 nm with 435 nm excitation was measured using a microplate reader Genios Pro (Tecan Japan Co., Ltd.). Fig. 13 shows the value after substraction of the measured value when V_{H}-cpBLA-V_{L} protein solution was not added as a background. As observed from Fig.13, a significant signal increase was observed in the sample added with ICP compared with samples ("TCP" and "PBS") without any ICP added. In other words, the increase of the ICP-dependent β-lactamase activity was observed.

### Example 12

### [Examples of detecting peptide using the anti-peptide antibody-enzyme fusion protein]

In order to confirm the general versatility of the antigen detection system using the fusion protein (antibody-enzyme fusion protein) of the present invention, a detection system using the antibody-enzyme fusion protein having an antibody Fv different from the above-mentioned Fv was constructed, and it was tested whether similar changes in the antigen-dependent enzyme activity could be observed. Specifically, an experiment for detecting an antigen peptide was performed using the antibody-enzyme fusion protein in which VH and VL genes in V_{H}-cpBLA-V_{L}-pET26 produced in Example 3 were replaced with the genes derived from the anti-osteocalcin C-terminal peptide antibody KTM219 (Anal. Chem. 79, 6197 (2007)). Specifically, this experiment was performed according to the following procedures.

First, in order to replace the VL gene of the V_{H}-cpBLA-V_{L}/pET26 and to remove the unnecessary XhoI site located in the 3' side, the VL gene of the KTM219 was amplified using the following primers. 100 ml of the reaction mixture was prepared with 50 pmol of MKback2A (SEQ ID NO: 23: GCGCAAGCTCAGTCGACGGAYATTGTGMTSACMCARWCTMCA) (the 12^{th} to 17^{th} nucleotide of the 5' -terminal is SalI site), 50 pmol of VkNotFor (SEQ ID NO: 24: ATGGTGCTCGACTGCGGCCGCCCGTTTTAT), 10 ng of pKST2 (KTM219; Anal. Chem. 79, 6197(2007)), 0.2 mM (final concentration of reaction solution) of dNTPs, 10 µl of 10x Ex-Taq buffer and 5 unit of Ex-Taq, and the PCR reaction was conducted at 94°C for 5 minutes, followed by 25 cycles of 94°C for 30 seconds, 65°C for 30 seconds, and 72°C for 1 minute, thereafter incubating at 72°C for 10 minutes. Further, the obtained reaction mixture was subjected to agarose gel electrophoresis for purification. Next, in a similar manner, the fragment containing a NotI-DraIII region was amplified with 50 pmol of NotdXback (SEQ ID NO: 25: GCAGTCGAGCACCATCACCACCACCAC), 50 pmol of pETDra3For (SEQ ID NO: 26: TGAGTGTTGTTCCAGTTTGG), and 10 ng of V_{H}-cpBLA-V_{L}/pET26. The obtained reaction mixture was purified similarly.

Overlap extension PCR was performed as in Example 1 using these purified DNA fragments, and Mkback2A and pETDra3For as primers. The obtained PCR products were purified, and subjected to restriction enzyme digestion with SalI and DrallI. After purification, the SalI-DraIII fragment was obtained. This SalI-DraIII fragment was inserted into V_{H}-cpBLA-V_{L}/pET26 also digested with SalI and DrallI and purified, and the plasmid was named as (ICP)-cpBLA-V_{L}(219)/pET26. After confirming the sequence of this plasmid, PCR reaction was conducted to amplify KTM219VH with primers M13RV (SEQ ID NO: 27: CAGGAAACAGCTATGAC) and JH1 (SEQ ID NO: 28: ACTGCTCGAGACGGTGACCGTGGTCCC), and pKST2 (KTM219) as a template. The obtained PCR product was subjected to a restriction enzyme digestion with NcoI and XhoI. After purification, the NcoI-XhoI fragment was obtained. This NcoI-XhoI fragment was inserted into V_{H}(ICP)-cpBLA-V_{L}(219)/pET26 also digested with NcoI and XhoI and purified, and the plasmid was named as V_{H}-cpBLA-V_{L}/pET26 (219).

### [Expression and purification of insoluble protein, and refolding thereof]

The BL21 (DE3, pLysS) strain was transformed with V_{H}-cpBLA-VL/pET26(219), and cultured at 30°C overnight on an LB agar medium plate containing 50 µg/ml of kanamycin and 37 µg/ml of chloramphenicol, to prepare the antibody-enzyme fusion protein expressing strain (V_{H}-cpBLA-V_{L}/pET26 (219) /BL21 strain). The colonies were picked with a toothpick, inoculated and cultured in a 4 ml of an LBKC medium at 37°C overnight. 250 µl of this pre-cultured solution was inoculated and cultured in 250 ml of the LBKC medium at 37°C overnight. When O.D.₆₀₀ reached around 0.6, IPTG with the final concentration of 1 mM was added. After further incubation at 37°C overnight, the culture solution was centrifuged at 6000 g for 10 minutes at 4°C, and collected. The cells were suspended in 25 ml of TALON Buffer, and disrupted by ultrasonic treatment on ice. The DNA was eliminated by adding 2.5 µl of Benzonase (Novagen Inc.) and MgCl₂ with the final concentration of 1 mM and incubating for 30 minutes on ice. After centrifugation at 6000 g for 10 minutes, the supernatant was discarded, 25 ml of 1% TritonX-100 and 1 mM EDTA were added to the remaining pellets and vortexed. The insoluble fractions were washed by conducting 3 times the centrifugation (6000 g, 10 minutes).

The washed precipites (insoluble fractions) were suspended in TALON Buffer containing 10 ml of 6 M guanidine hydrochloride (GdnHCL). 200 µl of TALON metal affinity column was added to this suspension, and the protein was purified by a batch/gravity-flow procedure under denaturation conditions according to the instructions. Proteins were eluted with elution buffer (200 mM Imidazole, 50 mM phosphate, 300 mM NaCl, 5.4 M GdnHCl, pH 7.0).

After measurement of the amount of the protein in an eluate by UV, 150 µl of the eluate (including about 320 µg of protein) was separated and added with 2 mercaptoethanol with a final concentration of 10 mM and EDTA with the final concentration of 1 mM. After being left at 30°C for 30 minutes, it was shifted to a Slide-A-Lyzer dialysis cassette (MW 10k cutoff, Pierce Biotechnology Inc.). Dialysis was performed with dialysis external solution (later mentioned dialysis external solutions 1 to 5) and the method described in J. Immunol. Methods 219, 119 (1998), and was conducted 5 times to 500 mL of dialysis external solution at 4°C for 24 hours each, and centrifuged at 4°C at 15k rpm for 10 minutes, and about 1.7 ml of the solubilized sample was obtained. This solubilized sample contained 3.7 µM of the antibody-enzyme fusion protein (V_{H}-cpBLA-V_{L}(219)).
Dialysis external solution 1: 50 mM TrisHCL, 3 M GdnHCL, 200 mM NaCl, 1 mM EDTA, pH 8.0;
Dialysis external solution 2: 50 mM TrisHCL, 2 M GdnHCL, 200 mM NaCl, 1 mM EDTA, pH 8.0;
Dialysis external solution 3: 50 mM TrisHCL, 1 M GdnHCL, 200 mM NaCl, 1 mM EDTA, 180 µM GSSG, 400 mM L-arginine, pH 8.0;
Dialysis external solution 4: 50 mM TrisHCL, 0.5 M GdnHCL, 200 mM NaCl, 1 mM EDTA, pH 8.0;
Dialysis external solution 5: 50 mM TrisHCL, 200 mM NaCl, 1 mM EDTA, pH 8.0;

### [Activity assay of the antibody-enzyme fusion protein]

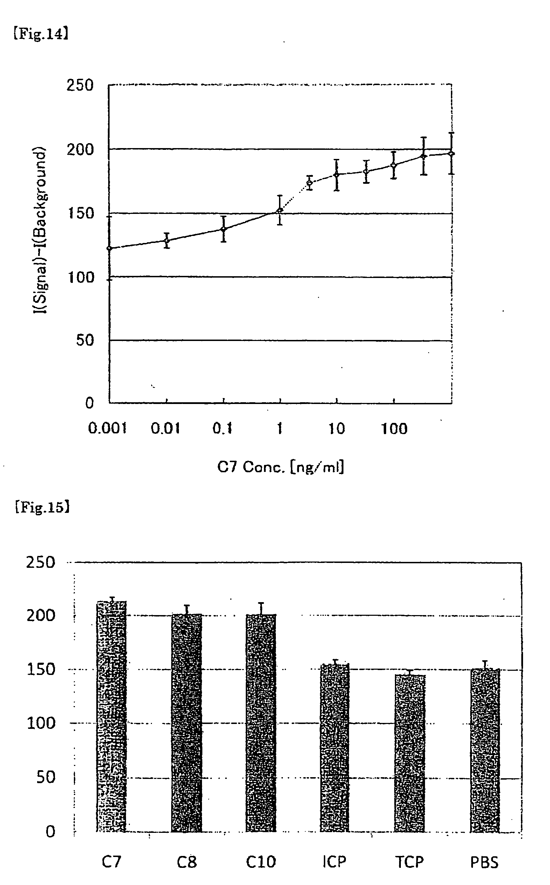

Activity of the above prepared antibody-enzyme fusion protein (V_{H}-cpBLA-V_{L}(219)) was assayed using Fluorocillin. The antigen peptide BGP-C7 at a final concentration of 0 - 2000 ng/ml and the antibody-enzyme fusion protein at a final concentration of 300 nM were mixed in 50 µl of PBS, and the mixture was dispensed into black microplate wells. After adding 50 µL of 2 µM Fluorocillin in PBS, the mixture was incubated at 28°C for 60 minutes, and read for its fluorescence intensity (intensity at 535 nm with excitation light irradiation at 485 nm). Also, control experiments were performed without antibody-enzyme fusion protein, and the fluorescence intensity of these wells was used for background subtraction. As a result, as shown in Fig.14, significant increase of the fluorescence intensity was observed by BGP-C7 in a dose-dependent manner. For information, the measurement of the present experiment was performed in triplicate. Black circles in Fig.14 indicate the average value of the measured values and the error bars indicate 1SD.

Further, similar experiments were performed using a substance other than BGP-C7 (BGP-C8, BGP-C10, ICP, TCP with each final concentration of 1 µg/ml) as antigens. The results are shown in Fig. 15. As observed from the results in Fig.15, whereas BGP-C8 and BGP-C10 which is detectable by open sandwich ELISA with KTM219 (Anal. Chem. 79, 6197 (2007) resulted in increase of the signal intensity, ICP and TCP which is not specifically recognized resulted in only the same signal intensity as PBS. For information, the measurement of the present experiments was conducted in triplicate. The values of the graph in Fig.15 indicate the average value of the measured values, and the error bars indicate 1SD.

The results from the above mentioned experiments showed versatility and specificity to the target antigen of the antigen detection system using the fusion protein (antibody-enzyme fusion protein) of the present invention.

### Industrial Applicability

According to the present invention, a target antigen even with a low molecular weight can be detected in a simpler, more efficient, more stable manner and with higher sensitivity. In addition, the method for detecting an antigen of the present invention, unlike the typical ELISA, is significantly advantageous over the conventional ELISA in that it is a measurement method (homogeneous assay) that can measure without any operation of washing labeled secondary antibodies (in non-competitive assays) or labeled antigens (in competitive assays) that have not been bound and that automation of the measuring device is easy, enabling a speedy measurement (without requiring any skill with simple operation). Therefore, the present invention can be usefully adapted to an immunoassay system such as in clinical diagnosis of diseases, test of food products, environmental analysis and the like.

## Claims

1. A fusion protein comprised of a polypeptide containing a V_{H} domain of an antibody; a given protein; a linker peptide; a partner protein having a binding activity to the protein and capable of detecting the presence or absence of the binding to the protein; and a polypeptide containing a V_{L} domain of the antibody; in this order or in reverse order, wherein the presence or absence of the binding of the polypeptide containing a V_{H} domain and the polypeptide containing a V_{L} domain to the antigen induces the presence or absence of the binding of the protein and the partner protein, wherein the combination of the given protein and the partner protein is a combination of a protein containing an N-terminal fragment of an enzyme protein and a protein containing a C-terminal fragment of the enzyme protein, and wherein the presence or absence of the binding of the protein containing the N-terminal fragment and the protein containing the C-terminal fragment can be detected by the change of a given enzyme activity of the enzyme protein.

2. The fusion protein according to claim 1, wherein the enzyme protein is β-lactamase.

3. The fusion protein according to claim 2, wherein the protein containing an N-terminal fragment of β-lactamase is a protein consisted of the amino acid sequence represented by SEQ ID NO: 5, and the protein containing a C-terminal fragment of β-lactamase is a protein consisted of the amino acid sequence represented by SEQ ID NO: 7.

4. The fusion protein according to any one of claims 1 to 3, wherein the linker peptide is the amino acid sequence consisted of Asp-Lys-Ser.

5. The fusion protein according to any one of claims 1 to 4, wherein the sequence of a given protein; a linker peptide; and a partner protein is a protein consisted of the amino acid sequence represented by SEQ ID NO: 13.

6. A DNA encoding the fusion protein according to any one of claims 1 to 5.

7. A recombinant vector having the DNA according to claim 6.

8. A transformed cell transformed by the recombinant vector according to claim 7.

9. A method for detecting an antigen in a sample, comprising the steps of: contacting the sample with the fusion protein according to any one of claims 1 to 5 in which a polypeptide containing a V_{H} domain and a polypeptide containing a V_{L} domain can bind to the antigen, or with the transformed cell according to claim 8; and detecting the binding of a partner protein and a given protein in the fusion protein or in a fusion protein which is expressed in the transformed cell.

10. A kit for detecting an antigen provided with the fusion protein according to any one of claims 1 to 5 which is a protein wherein a given enzyme activity exhibited by a given protein and/or a partner protein changes when the given protein and the partner protein are bound to each other compared to when they are not bound, or the transformed cell according to claim 8; and a chromogenic substrate or a fluorogenic substrate that can be detected due to the change of the enzyme activity.

## Patentansprüche

1. Fusionsprotein, umfassend ein Polypeptid, das eine V_{H}-Domäne eines Antikörpers enthält; ein bestimmtes Protein; ein Linkerpeptid; ein Partnerprotein, das eine Bindungsaktivität zu dem Protein aufweist und das das Vorhandensein oder das Fehlen der Bindung an das Protein nachweisen kann; und ein Polypeptid, das eine V_{L}-Domäne des Antikörpers enthält; und zwar in dieser Reihenfolge oder in umgekehrter Reihenfolge, wobei das Vorhandensein oder das Fehlen der Bindung des Polypeptids, das eine V_{H}-Domäne enthält, und des Polypeptids, das eine V_{L}-Domäne enthält, an das Antigen das Vorhandensein oder das Fehlen der Bindung des Proteins und des Partnerproteins induziert, wobei die Kombination aus dem bestimmten Protein und dem Partnerprotein eine Kombination aus einem Protein, das ein N-terminales Fragment eines Enzymproteins enthält, und einem Protein ist, das ein C-terminales Fragment des Enzymproteins enthält, und wobei das Vorhandensein oder das Fehlen der Bindung des Proteins, das das N-terminale Fragment enthält, und des Proteins, das das C-terminale Fragment enthält, durch die Änderung einer bestimmten Enzymaktivität des Enzymproteins nachgewiesen werden kann.

2. Fusionsprotein nach Anspruch 1, wobei das Enzymprotein β-Lactamase ist.

3. Fusionsprotein nach Anspruch 2, wobei das Protein, das ein N-terminales Fragment der β-Lactamase enthält, ein Protein ist, das aus der Aminosäuresequenz gemäß SEQ ID NO: 5 besteht, und das Protein, das ein C-terminales Fragment der β-Lactamase enthält, ein Protein ist, das aus der Aminosäuresequenz gemäß SEQ ID NO: 7 besteht.

4. Fusionsprotein nach einem der Ansprüche 1 bis 3, wobei das Linkerpeptid die Aminosäuresequenz ist, die aus Asp-Lys-Ser besteht.

5. Fusionsprotein nach einem der Ansprüche 1 bis 4, wobei die Sequenz eines bestimmten Proteins; eines Linkerpeptids; und eines Partnerproteins ein Protein ist, das aus der Aminosäuresequenz gemäß SEQ ID NO: 13 besteht.

6. DNA, die das Fusionsprotein nach einem der Ansprüche 1 bis 5 codiert.

7. Rekombinanter Vektor mit der DNA nach Anspruch 6.

8. Transformierte Zelle, die durch den rekombinanten Vektor nach Anspruch 7 transformiert wurde.

9. Verfahren zum Nachweisen eines Antigens in einer Probe, umfassend die Schritte: Zusammenbringen der Probe mit dem Fusionsprotein nach einem der Ansprüche 1 bis 5, wobei ein Polypeptid, das eine V_{H}-Domäne enthält, und ein Polypeptid, das eine V_{L}-Domäne enthält, an das Antigen binden können, oder mit der transformierten Zelle nach Anspruch 8; und Nachweisen der Bindung eines Partnerproteins und eines bestimmten Proteins in dem Fusionsprotein oder in einem Fusionsprotein, das in der transformierten Zelle exprimiert wird.

10. Kit zum Nachweisen eines Antigens, das ausgestattet ist mit dem Fusionsprotein nach einem der Ansprüche 1 bis 5, wobei es sich um ein Protein handelt, bei dem sich eine bestimmte Enzymaktivität eines bestimmten Proteins und/oder Partnerproteins verändert, wenn das bestimmte Protein und das Partnerprotein miteinander verbunden sind, im Vergleich zu dem Fall, wenn sie nicht verbunden sind, oder mit der transformierten Zelle nach Anspruch 8; und einem chromogenen Substrat oder einem fluorogenen Substrat, das aufgrund der Änderung der Enzymaktivität nachgewiesen werden kann.

## Revendications

1. Protéine de fusion constituée d'un polypeptide contenant un domaine V_{H} d'un anticorps ; une protéine donnée ; un peptide lieur ; une protéine partenaire ayant une activité de liaison à la protéine et capable de détecter la présence ou l'absence de la liaison à la protéine ; et un polypeptide contenant un domaine V_{L} de l'anticorps ; dans cet ordre ou dans l'ordre inverse, la présence ou l'absence de la liaison du polypeptide contenant un domaine V_{H} et du polypeptide contenant un domaine V_{L} à l'antigène induisant la présence ou l'absence de la liaison de la protéine et la protéine partenaire, la combinaison de la protéine donnée et de la protéine partenaire étant une combinaison d'une protéine contenant un fragment N-terminal d'une protéine enzymatique et d'une protéine contenant un fragment C-terminal de la protéine enzymatique, et la présence ou l'absence de la liaison de la protéine contenant le fragment N-terminal et de la protéine contenant le fragment C-terminal pouvant être détectée par le changement d'une activité enzymatique donnée de la protéine enzymatique.

2. Protéine de fusion selon la revendication 1, la protéine enzymatique étant la β-lactamase.

3. Protéine de fusion selon la revendication 2, la protéine contenant un fragment N-terminal de β-lactamase étant une protéine constituée de la séquence d'acides aminés représentée par SEQ ID NO: 5, et la protéine contenant un fragment C-terminal de β-lactamase étant une protéine constituée de la séquence d'acides aminés représentée par SEQ ID NO: 7.

4. Protéine de fusion selon l'une quelconque des revendications 1 à 3, le peptide lieur étant la séquence d'acides aminés constituée de Asp-Lys-Ser.

5. Protéine de fusion selon l'une quelconque des revendications 1 à 4, la séquence d'une protéine donnée ; un peptide lieur ; et une protéine partenaire étant une protéine constituée de la séquence d'acides aminés représentée par SEQ ID NO: 13.

6. ADN codant pour la protéine de fusion selon l'une quelconque des revendications 1 à 5.

7. Vecteur recombinant ayant l'ADN selon la revendication 6.

8. Cellule transformée transformée par le vecteur recombinant selon la revendication 7.

9. Procédé pour détecter un antigène dans un échantillon, comprenant les étapes de : mise en contact de l'échantillon avec la protéine de fusion selon l'une quelconque des revendications 1 à 5 où un polypeptide contenant un domaine V_{H} et un polypeptide contenant un domaine V_{L} peuvent se lier à l'antigène, ou avec la cellule transformée selon la revendication 8 ; et détection de la liaison d'une protéine partenaire et d'une protéine donnée dans la protéine de fusion ou dans une protéine de fusion qui est exprimée dans la cellule transformée.

10. Kit pour détecter un antigène fourni avec la protéine de fusion selon l'une quelconque des revendications 1 à 5 qui est une protéine, une activité enzymatique donnée présentée par une protéine donnée et/ou une protéine partenaire changeant lorsque la protéine donnée et la protéine partenaire sont mutuellement liées par rapport à la situation dans laquelle elles ne sont pas liées, ou la cellule transformée selon la revendication 8, et un substrat chromogène ou un substrat fluorogène qui peut être détecté en raison du changement de l'activité enzymatique.
